# EUROPEAN PATENT APPLICATION

(11) **EP 3 656 872 A1**
(43) Date of publication of application: **27.05.2020**
(21) Application number: 19186124.4
(22) Date of filing: 09.12.2016
(51) Int. Cl.: C12Q 1/6813

(54) **LABEL-FREE ANALYSIS OF RNA CAPPING EFFICIENCY USING RNASE H, PROBES AND LIQUID CHROMATOGRAPHY/MASS SPECTROMETRY**

(30) Priority: 09.12.2015 US 201562265099 P
(62) Divisional of application: 16820351.1
(71) Applicant: Novartis AG, 4056 Basel (CH)
(72) Inventor: BEVERLY, Michael, Cambridge, MA 02139 (US)
(74) Representative: Masselink, Johannes Hendrikus Bernardus

(57) **Abstract**

Methods to qualitatively and quantitatively determine mRNA capping, and to determine 5' capping efficiency and 5' cap identity in RNA samples, all without the need for radiolabels, by using tagged probes that are complementary to the 5' end of target RNA and RNAse H to cleave the 5' end of RNA, then using LC-MS to determine the 5' RNA products.

SEQUENCE LISTING

<110> NOVARTIS AG

<120> LABEL-FREE ANALYSIS OF RNA CAPPING EFFICIENCY USING RNASE H,
      PROBES AND LIQUID CHROMATOGRAPHY/MASS SPECTROMETRY

<130> PAT057158-WO-PCT

<140> PCT/IB2016/057499
<141> 2016-12-09

<150> 62/265,099
<151> 2015-12-09

<160> 24

<170> PatentIn version 3.5

<210> 1
<211> 22
<212> RNA
<213> Artificial Sequence

<220>
<221> source
<223> /note="Description of Artificial Sequence: Synthetic
      oligonucleotide"


<220>
<221> source
<223> /note="5' Cap0 (m7Gppp) modified"

<400> 1
gggagacgcg uguuaaauaa ca                                                22


<210> 2
<211> 22
<212> RNA
<213> Artificial Sequence

<220>
<221> source
<223> /note="Description of Artificial Sequence: Synthetic
      oligonucleotide"


<220>
<221> source
<223> /note="5' uncapped triphosphate (ppp) modified"

<400> 2
gggagacgcg uguuaaauaa ca                                                22


<210> 3
<211> 22
<212> RNA
<213> Artificial Sequence

<220>

<221> source
<223> /note="Description of Artificial Sequence: Synthetic
      oligonucleotide"


<220>
<221> source
<223> /note="5' uncapped diphosphate (pp) modified"

<400> 3
gggagacgcg uguuaaauaa ca                                                22


<210> 4
<211> 22
<212> RNA
<213> Artificial Sequence

<220>
<221> source
<223> /note="Description of Artificial Sequence: Synthetic
      oligonucleotide"


<220>
<221> source
<223> /note="5' uncapped monophosphate (p) modified"

<400> 4
gggagacgcg uguuaaauaa ca                                                22


<210> 5
<211> 22
<212> RNA
<213> Artificial Sequence

<220>
<221> source
<223> /note="Description of Artificial Sequence: Synthetic
      oligonucleotide"


<220>
<221> source
<223> /note="5' Rev cap (pm7) modified"

<400> 5
gggagacgcg uguuaaauaa ca                                                22


<210> 6
<211> 22
<212> RNA
<213> Artificial Sequence

<220>
<221> source
<223> /note="Description of Artificial Sequence: Synthetic
      oligonucleotide"


<220>

<221> source
<223> /note="5' Cap1 (m7Gpppm) modified"

<400> 6
gggagacgcg uguuaaauaa ca                                                22


<210> 7
<211> 22
<212> RNA
<213> Artificial Sequence

<220>
<221> source
<223> /note="Description of Artificial Sequence: Synthetic
      oligonucleotide"


<220>
<221> source
<223> /note="5' Cap1 triphosphate (pppm) modified"

<400> 7
gggagacgcg uguuaaauaa ca                                                22


<210> 8
<211> 22
<212> RNA
<213> Artificial Sequence

<220>
<221> source
<223> /note="Description of Artificial Sequence: Synthetic
      oligonucleotide"


<220>
<221> source
<223> /note="5' Cap1 diphosphate (ppm) modified"

<400> 8
gggagacgcg uguuaaauaa ca                                                22


<210> 9
<211> 22
<212> RNA
<213> Artificial Sequence

<220>
<221> source
<223> /note="Description of Artificial Sequence: Synthetic
      oligonucleotide"


<220>
<221> source
<223> /note="5' Cap1 monophosphate (pm) modified"

<400> 9
gggagacgcg uguuaaauaa ca                                                22

<210> 10
<211> 22
<212> RNA
<213> Artificial Sequence

<220>
<221> source
<223> /note="Description of Artificial Sequence: Synthetic
      oligonucleotide"


<220>
<221> source
<223> /note="5' G cap (Gppp) modified"

<400> 10
gggagacgcg uguuaaauaa ca                                                22


<210> 11
<211> 22
<212> RNA
<213> Artificial Sequence

<220>
<221> source
<223> /note="Description of Artificial Sequence: Synthetic
      oligonucleotide"


<220>
<221> source
<223> /note="5' Cap0 (m7Gppp) modified"

<220>
<221> modified_base
<222> (7)..(7)
<223> 5-methylcytidine

<220>
<221> modified_base
<222> (9)..(9)
<223> 5-methylcytidine

<220>
<221> modified_base
<222> (21)..(21)
<223> 5-methylcytidine

<400> 11
gggagacgcg uguuaaauaa ca                                                22


<210> 12
<211> 22
<212> RNA
<213> Artificial Sequence

<220>
<221> source
<223> /note="Description of Artificial Sequence: Synthetic
      oligonucleotide"

<220>
<221> source
<223> /note="5′ uncapped triphosphate (ppp) modified"

<220>
<221> modified_base
<222> (7)..(7)
<223> 5-methylcytidine

<220>
<221> modified_base
<222> (9)..(9)
<223> 5-methylcytidine

<220>
<221> modified_base
<222> (21)..(21)
<223> 5-methylcytidine

<400> 12
gggagacgcg uguuaaauaa ca                                                22


<210> 13
<211> 22
<212> RNA
<213> Artificial Sequence

<220>
<221> source
<223> /note="Description of Artificial Sequence: Synthetic
      oligonucleotide"


<220>
<221> source
<223> /note="5′ uncapped diphosphate (pp) modified"

<220>
<221> modified_base
<222> (7)..(7)
<223> 5-methylcytidine

<220>
<221> modified_base
<222> (9)..(9)
<223> 5-methylcytidine

<220>
<221> modified_base
<222> (21)..(21)
<223> 5-methylcytidine

<400> 13
gggagacgcg uguuaaauaa ca                                                22


<210> 14
<211> 22
<212> RNA
<213> Artificial Sequence

<220>
<221> source
<223> /note="Description of Artificial Sequence: Synthetic
      oligonucleotide"

<220>
<221> source
<223> /note="5' G cap (Gppp) modified"

<220>
<221> modified_base
<222> (7)..(7)
<223> 5-methylcytidine

<220>
<221> modified_base
<222> (9)..(9)
<223> 5-methylcytidine

<220>
<221> modified_base
<222> (21)..(21)
<223> 5-methylcytidine

<400> 14
gggagacgcg uguuaaauaa ca                                                22

<210> 15
<211> 24
<212> DNA
<213> Artificial Sequence

<220>
<221> source
<223> /note="Description of Artificial Sequence: Synthetic
      cleavage probe"

<220>
<221> source
<223> /note="Description of Combined DNA/RNA Molecule: Synthetic
      cleavage probe"

<220>
<221> misc_feature
<222> (7)..(24)
<223> /note="2' O-methyl modified base"

<220>
<221> source
<223> /note="3' 3BioTEG modified"

<400> 15
tttgttauuu aacacgcguc uccc                                              24

<210> 16
<211> 48
<212> RNA
<213> Artificial Sequence

<220>
<221> source
<223> /note="Description of Artificial Sequence: Synthetic
      oligonucleotide"

<400> 16
gggagacgcg uguuaaauaa caaaucucaa cacaacauau acaaaaca                48


<210> 17
<211> 2387
<212> RNA
<213> Photinus pyralis

<400> 17
cugcagaaau aacuagguac uaagcccguu ugugaaaagu ggccaaaccc auaaauuugg      60

caauuacaau aaagaagcua aaauuguggu caaacucaca aacauuuuua uuauauacau     120

uuuaguagcu gaugcuuaua aaagcaauau uuaaaucgua aacaacaaau aaaauaaaau     180

uuaaacgaug ugauuaagag ccaaaggucc ucuagaaaaa gguauuuaag caacggaauu     240

ccuuuguguu acauucuuga augucgcucg cagugacauu agcauuccgg uacuguuggu     300

aaaauggaag acgccaaaaa cauaaagaaa ggcccggcgc cauucuaucc ucuagaggau     360

ggaaccgcug gagagcaacu gcauaaggcu augaagagau acgcccuggu uccuggaaca     420

auugcuuuug ugaguauuuc ugucugauuu cuuucgaguu aacgaaaugu ucuuauguuu     480

cuuuagacag augcacauau cgaggugaac aucacguacg cggaauacuu cgaaaugucc     540

guucgguugg cagaagcuau gaaacgauau gggcugaaua caaaucacag aaucgucgua     600

ugcagugaaa acucucuuca auucuuuaug ccgguguugg gcgcguuauu uaucggaguu     660

gcaguugcgc ccgcgaacga cauuuauaau gaacguaagc acccucgcca ucagaccaaa     720

gggaaugacg uauuuaauuu uuaaggugaa uugcucaaca guaugaacau uucgcagccu     780

accguagugu uuguuuccaa aaagggguug caaaaaauuu ugaacgugca aaaaaaauua     840

ccaauaaucc agaaaauuau uaucauggau ucuaaaacgg auuaccaggg auuucagucg     900

auguacacgu ucgucacauc ucaucuaccu cccgguuuua augaauacga uuuuguacca     960

gaguccuuug aucgugacaa aacaauugca cugauaauga auuccucugg aucuacuggg    1020

uuaccuaagg guguggcccu uccgcauaga acugccugcg ucagauucuc gcaugccagg    1080

uaugucguau aacaagagau uaaguaaugu ugcuacacac auuguagaga uccuauuuuu    1140

ggcaaucaaa ucauuccgga uacugcgauu uuaaguguug uuccauucca ucacgguuuu    1200

ggaauguuua cuacacucgg auauuugaua uguggauuuc gagucgucuu aauguauaga    1260

uuugaagaag agcuguuuuu acgaucccuu caggauuaca aaauucaaag ugcguugcua    1320

guaccaaccc uauuuucauu cuucgccaaa agcacucuga uugacaaaua cgauuuaucu    1380

aauuuacacg aaauugcuuc ugggggcgca ccucuuucga aagaagucgg ggaagcgguu    1440

gcaaaacggu gaguuaagcg cauugcuagu auuucaaggc ucuaaaacgg cgcguagcuu 1500

ccaucuucca gggauacgac aaggauaugg gcucacugag acuacaucag cuauucugau 1560

uacacccgag gggaugauaa aaccgggcgc ggucgguaaa guuguuccau uuuuugaagc 1620

gaagguugug gaucuggaua ccgggaaaac gcugggcguu aaucagagag gcgaauuaug 1680

ugucagagga ccuaugauua uguccgguua uguaaacaau ccggaagcga ccaacgccuu 1740

gauugacaag gauggauggc uacauucugg agacauagcu uacugggacg aagacgaaca 1800

cuucuucaua guugaccgcu ugaagucuuu aauuaaauac aaaggauauc agguaaugaa 1860

gauuuuuaca ugcacacacg cuacaauacc uguagguggc ccccgcugaa uuggaaucga 1920

uauuguuaca acaccccaac aucuucgacg cgggcguggc aggucuuccc gacgaugacg 1980

ccggugaacu ucccgccgcc guuguuguuu uggagcacgg aaagacgaug acggaaaaag 2040

agaucgugga uuacgucgcc aguaaaugaa uucguuuuac guuacucgua cuacaauucu 2100

uuucauaggu caaguaacaa ccgcgaaaaa guugcgcgga ggaguugugu uuguggacga 2160

aguaccgaaa ggucuuaccg gaaaacucga cgcaagaaaa aucagagaga uccucauaaa 2220

ggccaagaag ggcggaaagu ccaaauugua aaauguaacu guauucagcg augacgaaau 2280

ucuuagcuau uguaauauua uaugcaaauu gaugaauggu aauuuuguaa uuguggguca 2340

cuguacuauu uuaacgaaua auaaaaucag guauagguaa cuaaaaa 2387

<210> 18
<211> 24
<212> DNA
<213> Artificial Sequence

<220>
<221> source
<223> /note="Description of Artificial Sequence: Synthetic
      cleavage probe"

<220>
<221> source
<223> /note="Description of Combined DNA/RNA Molecule: Synthetic
      cleavage probe"

<400> 18
tttgttauuu aacacgcguc uccc 24

<210> 19
<211> 28
<212> RNA
<213> Artificial Sequence

<220>
<221> source
<223> /note="Description of Artificial Sequence: Synthetic
      oligonucleotide"

<220>
<221> source
<223> /note="5' 7mGppp modified"

<400> 19
gggagacgcg uguuaaauaa caaaucuc                                          28


<210> 20
<211> 22
<212> RNA
<213> Artificial Sequence

<220>
<221> source
<223> /note="Description of Artificial Sequence: Synthetic
      oligonucleotide"


<220>
<221> source
<223> /note="5' 7mGppp modified"

<400> 20
gggagacgcg uguuaaauaa ca                                                22


<210> 21
<211> 22
<212> DNA
<213> Artificial Sequence

<220>
<221> source
<223> /note="Description of Artificial Sequence: Synthetic
      oligonucleotide"


<220>
<221> source
<223> /note="5' 7mGppp modified"

<400> 21
gggagacgcg tgttaaataa ca                                                22


<210> 22
<211> 23
<212> DNA
<213> Artificial Sequence

<220>
<221> source
<223> /note="Description of Artificial Sequence: Synthetic
      oligonucleotide"


<220>
<221> source
<223> /note="5' 7mGppp modified"

<400> 22

gggagacgcg tgttaaataa caa                                              23

<210> 23
<211> 23
<212> DNA
<213> Artificial Sequence

<220>
<221> source
<223> /note="Description of Artificial Sequence: Synthetic
      oligonucleotide"

<220>
<221> source
<223> /note="5′ p modified"

<400> 23
gggagacgcg tgttaaataa caa                                              23

<210> 24
<211> 23
<212> DNA
<213> Artificial Sequence

<220>
<221> source
<223> /note="Description of Artificial Sequence: Synthetic
      oligonucleotide"

<220>
<221> source
<223> /note="5′ p7m modified"

<400> 24
gggagacgcg tgttaaataa caa                                              23

## Description

### FIELD OF THE INVENTION

The invention relates generally to modified forms of RNA not used in recombinant technology and to measuring processes involving enzymes, with the release of a bound marker.

### BACKGROUND OF THE INVENTION

The growing interest in developing mRNA for therapeutic purposes will require a thorough characterization of the mRNA, including the 5' cap. Thess A et al., Mol. Ther. 23(9): 1456-1464 (2015); Weissman D et al., Mol. Ther. 23(9): 1416-1417 (2015). Such a development will need methods that are robust, accurate, and able to process many samples quickly.

Previous methods for characterizing the 5' cap have used enzymatic digestion of the mRNA into mononucleotides, followed by polyacrylamide gel electrophoresis (PAGE) or anion exchange high performance liquid chromatography (HPLC) of the cleavage products, along with radiolabel detection of P³² incorporated as the 5' terminal phosphate. Cleavage of the mRNA with RNAse T2 permitted a determination of capping efficiency. Using RNase T2 digestion followed by tobacco acid pyrophosphatase has provided information on cap orientation. Pasquinelli AE et al., RNA 1(9): 957-967 (1995); Peng ZH et al., Org. Lett. 4(2):161-164 (2002); Stepinski J et al., RNA 7 (10):1486-1495 (2001); Jemielity J et al., RNA 9(9): 1108-1122 (2003); Jemielity J et al., Nucleosides Nucleotides Nucleic Acids 22(5-8): 691-694 (2003); Grudzien E et al., RNA 10(9): 1479-1487 (2004). This combination of enzymes produced either pGp (if the cap is in the forward or correct orientation) or pm7Gp (if in the reverse orientation). These two species were then separated by HPLC and quantified by measuring P³².

Capping efficiency of the reaction has also been determined using sequence specific RNAse H probes to cleave off small sections from the 5' end of the mRNA and resolving the uncapped triphosphate from the m7GppG capped mRNA using PAGE with radiolabeled detection. Peng ZH et al., Org. Lett. 4(2):161-164 (2002); Tcherepanova IY et al., BMC Mol. Biol. 9: 90 (2008).

Regarding other methods, published PCT application WO 2012/135805 (Moderna Therapeutics) disclosed the detection of capped mRNA by LC-MS, corresponding to capping reaction efficiency. Published PCT application WO 2014/152659 (Shire Human Genetic Therapies) discloses and claims a method of quantifying mRNA capping efficiency by chromatography. WO2015101416 (Curevac GmbH) discloses and claims a method for analyzing RNA having a cleavage site for a catalytic nucleic acid molecule, in which the amount of capped RNA can be measured by quantitative mass spectroscopy. Lapham & Crothers, RNA 2(3): 289-296 (1996) disclosed a method of cleaving short sequences from the 5' end of the mRNA at a specific location.

While prior art methods provided information on the cap structure and orientation, they are limited to radiolabeled mRNA. These methods are also limited to the identification of different cap species based on the chromatographic resolution and retention time of standards. As research into using mRNA for therapeutic purposes increases, there is a need in the biotechnological arts for characterizing *in vitro* transcribed (IVT) synthesized mRNA. Sahin U et al., Nat. Rev. Drug Discov. 13(10):759-780 (2014); Geall AJ et al., Semin. Immunol. 25(2):152-159 (2013).

### SUMMARY OF THE INVENTION

In a first embodiment, the invention provides a radiolabel free method for identifying a 5' end cap on a target ribonucleic acid (RNA), comprising the steps of: (a) hybridizing a nonradiolabeled tagged probe to the target RNA, wherein the nucleotide sequence of the nonradiolabeled tagged probe is complementary to the 5' end of the target RNA, thus forming a duplex polynucleotide; (b) treating the duplex polynucleotide with RNAse H, thus cleaving the 5' end of the target RNA and forming a duplex polynucleotide containing the 5' end of the target RNA; (c) isolating the duplex polynucleotide, using a surface coated substrate that is coated with a reagent that binds to the nonradiolabeled tagged probe; (d) removing the duplex polynucleotide from the surface coated substrate; (e) denaturing the duplex polynucleotide, thus producing a single-stranded fragment of the 5' end of the target RNA and the nonradiolabeled tagged probe; (f) isolating the single-stranded fragment of the 5' end of the target RNA; (g) analyzing the single-stranded fragment of the 5' end of the target RNA by liquid chromatography/mass spectrometry (LC-MS); and (h) identifying the 5' end cap. In one embodiment, the target RNA is a messenger RNA (mRNA). In a second embodiment, the target RNA is a eukaryotic messenger RNA.

The method of the invention provides those of skill in the biotechnological arts with a way of identifying the 5' cap without the need for radiolabels, by using liquid chromatography coupled to electrospray mass spectrometry (LC-MS) and detecting their differences in mass and retention time. Using mass spectrometry as the detector allows identification based on an intrinsic property of the species that can be measured accurately and with high-resolution. Unambiguous identification of 5' cap cleavage products by mass spectrometry also enables identification of a variety of uncapped species.

In a third embodiment, the substrate of the surface coated substrate is magnetic beads. In a fourth embodiment, the reagent on the surface coated substrate that binds to the nonradiolabeled tagged probe is (a) streptavidin, where the nonradiolabeled tag is biotin; (b) avidin, where the nonradiolabeled tag is biotin; (c) an anti-biotin antibody, where the nonradiolabeled tag is biotin; (d) an anti-digoxigenin antibody, where the nonradiolabeled tag is digoxigenin; or (e) an anti-peptide antibody, where the nonradiolabeled tag is a peptide. In a fifth embodiment, the target RNA is synthesized *in vitro.*

In a sixth embodiment, the method of identification further comprises determining quantitative and qualitative information on the 5' cap from the unique mass of the single-stranded fragment of the 5' end of the target RNA. The method of the invention can qualitatively and quantitatively determine mRNA capping, 5' capping efficiency and 5' cap identity in mRNA samples.

In a seventh embodiment, the also provides a radiolabel free method for determining the 5' end orientation on target RNA, comprising the steps of: (a) hybridizing a nonradiolabeled tagged probe to the target RNA to form a partially duplex polynucleotide, wherein the nucleotide sequence of the nonradiolabeled tagged probe is complementary to the 5' end of the target RNA; (b) treating the duplex polynucleotide with RNAse H, to cleave the 5' end of the RNA and to form a duplex polynucleotide; (c) isolating the duplex polynucleotide containing the 5' end of the target RNA, using magnetic beads coated with a reagent that binds to the nonradiolabeled tagged probe; (d) treating the duplex polynucleotide with 5' RNA pyrophosphohydrolase (RppH); (e) removing the duplex polynucleotide from the magnetic beads; (f) denaturing the duplex polynucleotide, to produce a single-stranded fragment of the 5' end of the target RNA and the nonradiolabeled tagged probe; (g) isolating the single-stranded fragment of the 5' end of the target RNA; (h) analyzing the single-stranded fragment of the 5' end of the RNA by liquid chromatography/mass spectrometry (LC-MS); and (i) detecting the mass difference in the product to determine the orientation of the 5' cap.

The method of the invention was used to straightforwardly assay for cap orientation. Combining the RNAse H cleavage probe with a 5' RNA pyrophosphohydrolase (RppH) allowed for determination of capping orientation, which can be helpful when working with methods that co-transcriptionally cap mRNA.

In an eighth embodiment, the target RNA is a messenger RNA (mRNA). In a ninth embodiment, the target RNA is a eukaryotic messenger RNA.

In a tenth embodiment, the 5' end cap has a structure of formula I: 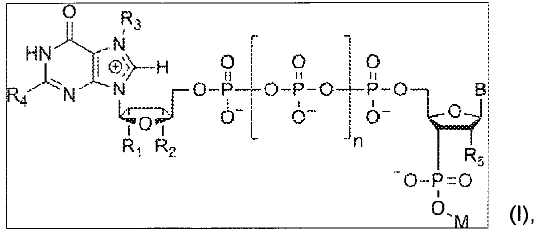 where B is a nucleobase; R₁ is selected from a halogen, OH, and OCH₃; R₂ is selected from H, OH, and OCH₃; R₃ is CH₃, CH₂CH₃, CH₂CH₂CH₃ or void; R₄ is NH₂; R₅ is selected from OH, OCH₃ or a halogen; n is 1, 2, or 3, or a phosphorothioate; and M is a nucleotide of the mRNA. In an eleventh, more specific embodiment, the nucleobase is guanine.

In a twelfth embodiment, the 5' end cap is a m⁷G cap with a structure of formula II: 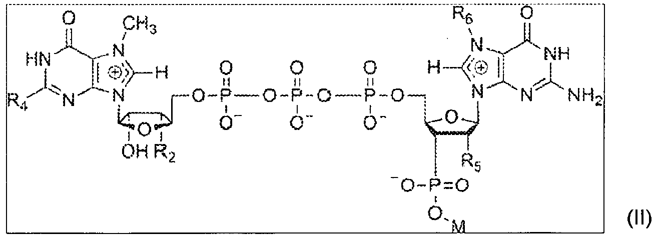 where, R₂ is H or CH₃; R₄ is NH₂; R₆ is OH or OCH₃; and M is a nucleotide of the RNA; or (in a thirteenth embodiment) an unmethylated cap with a structure of formula III 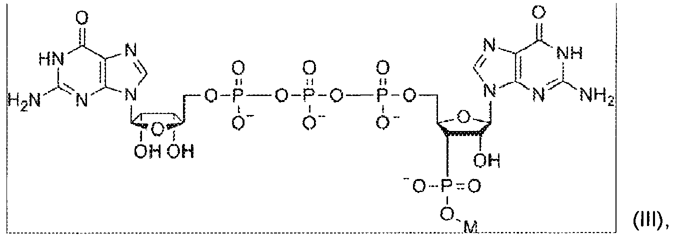 where M is a nucleotide of the RNA.

In a fourteenth embodiment, the target RNA is synthesized *in vitro.* In a fifteenth, more specific embodiment, the *in vitro* synthesized RNA contains one or more nucleotides selected from Ψ (pseudouridine); m⁵C (5-methylcytidine); m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms² m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f⁵C (5-formylcytidine); m⁵ Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m² ₂G (N²,N²-dimethylguanosine); m²Gm (N²,2'-O-dimethylguanosine); m²₂Gm (N²,N²2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine); cmnm⁵s2U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N³,2'-O-dimethyladenosine); m⁶ ₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); andac⁶A (N⁶-acetyladenosine). In a sixteenth, specific embodiment, the *in vitro* synthesized RNA contains one or more nucleotides selected from Ψ (pseudouridine), m⁵C (5-methylcytidine) or both.

In a seventeenth embodiment, the substrate of the surface coated substrate is magnetic beads. In another embodiment, the reagent on the surface coated substrate that binds to the nonradiolabeled tagged probe is: (a) streptavidin, wherein the nonradiolabeled tag is biotin; (b) avidin, wherein the nonradiolabeled tag is biotin; (c) an anti-biotin antibody, wherein the nonradiolabeled tag is biotin; (d) an anti-digoxigenin antibody, wherein the nonradiolabeled tag is digoxigenin; or (e) an anti-peptide antibody, wherein the nonradiolabeled tag is a peptide.

In an eighteenth embodiment, the invention further provides a radiolabel free method for determining capping efficiency, comprising the steps of: (a) providing an RNA sample comprising capped RNA and uncapped RNA; (b) hybridizing a nonradiolabeled tagged probe to the target RNA to form a duplex polynucleotide, wherein the nucleotide sequence of the nonradiolabeled tagged probe is complementary to the 5' end of the target RNA; (c) treating the duplex polynucleotide with RNAse H, to cleave the 5' end of the RNA and to form a duplex polynucleotide; (d) isolating the duplex polynucleotide containing the 5' end of the target RNA, using magnetic beads coated with a reagent that binds to the nonradiolabeled tagged probe; (e) removing the duplex polynucleotide from the magnetic beads; (f) denaturing the duplex polynucleotide, to produce a single-stranded fragment of the 5' end of the target RNA and the nonradiolabeled tagged probe; (g) isolating the single-stranded fragment of the 5' end of the target RNA; (h) analyzing the single-stranded fragment of the 5' end of the RNA by liquid chromatography/mass spectrometry (LC-MS); and (i) determining relative amount of the capped and uncapped single-stranded fragments, thereby quantifying mRNA capping efficiency.

In a nineteenth embodiment, the method of the invention provides a way of differentiating capped from uncapped RNA without the need for radiolabels, by using liquid chromatography coupled to electrospray mass spectrometry (LC-MS) and detecting their differences in mass and retention time. In a twentieth embodiment, the LC-MS based approach described above is automated to run in a high-throughput format for analyzing larger numbers of samples.

The method of the invention can be advantageously applied to determine the capping efficiency of *in vitro* transcription (IVT) synthesized mRNAs. In a twenty-first embodiment, the method of the invention is used to analyze mRNA of 2.2K and 9K nucleotides in length that contained the modified nucleotides pseudouridine (Ψ) and 5-methylcytidine (m⁵C). The successful result from the use of this embodiment demonstrated the utility of the method of the invention to study mRNA capping and the functionality over a range of mRNA lengths and with common modification types. The assay response for uncapped mRNA was linear, allowing quantitative determination of uncapped species. Uncapped triphosphate mRNA of 2.2K bases in the presence of 100 picomoles capped mRNA could be detected over the tested range of 0.5 to 25%. The analysis of several batches of capped mRNA revealed capping efficiencies ranging from 88-98%, which appears to be consistent with the V*accinia* capping enzyme system. mRNA capped using the ARCA system was found to have a capping efficiency of 70%, which is in agreement with that reported by Grudzien E et al., RNA 10(9): 1479-1487 (2004).

In a twenty-second embodiment, the method of the invention is used to troubleshoot the IVT capping process. In the use of this embodiment, the presence of diphosphate in the capped material indicated that the initial triphosphatase that removes one phosphate from the uncapped triphosphate mRNA was working well, but that the guanyltransferase that adds GTP to the diphosphate needed improvement. Similarly, the presence of unmethylated G-capped mRNA indicated that the N7-methyltransferase needed adjustment.

In a twenty-third embodiment, the 5' end cap has a structure of formula I: 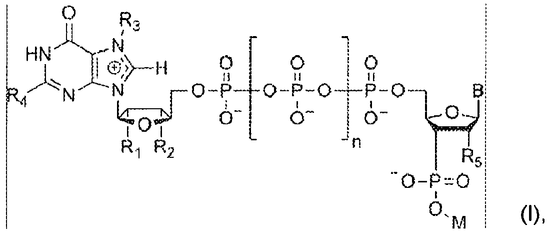 where B is a nucleobase; R₁ is selected from a halogen, OH, and OCH₃; R₂ is selected from H, OH, and OCH₃; R₃ is CH₃, CH₂CH₃, CH₂CH₂CH₃ or void; R₄ is NH₂; R₅ is selected from OH, OCH₃ or a halogen; n is 1, 2, or 3, or a phosphorothioate; and M is a nucleotide of the mRNA. In a twenty-fourth, more specific embodiment, the nucleobase is guanine.

In a twenty-fifth embodiment, the, the cap is a m⁷G cap with a structure of formula II: 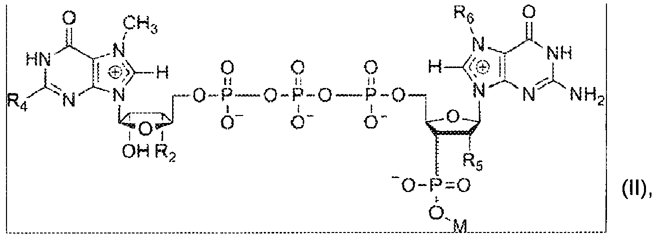 where R₂ is H or CH₃; R₄ is NH₂; R₆ is OH or OCH₃; and M is a nucleotide of the RNA; or (in a twenty-sixth embodiment) an unmethylated cap with a structure of formula III: 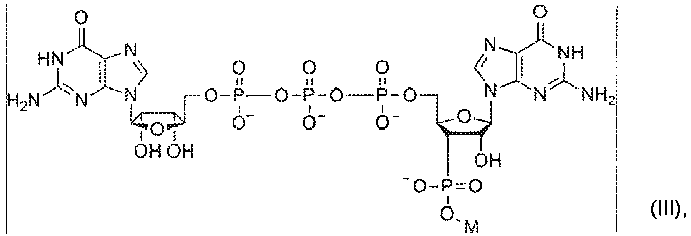 where M is a nucleotide of the RNA.

In a twenty-seventh embodiment, the RNA sample is an *in vitro* transcription (IVT) reaction mixture. In a twenty-eighth, more specific embodiment, the IVT reaction is a *Vaccinia* capped mRNA preparation. In a twenty-ninth embodiment, the *in vitro* synthesized RNA contains one or more nucleosides selected from Ψ (pseudouridine); m⁵C (5-methylcytidine); m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms² m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f⁵C (5-formylcytidine); m⁵ Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m² ₂G (N²,N²-dimethylguanosine); m²Gm (N²,2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁶U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁶Um (5-carboxymethylaminomethyl-2'-O-methyluridine); cmnm⁵s²U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N⁶,2'-O-dimethyladenosine); m⁶ ₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); andac⁶A (N⁶-acetyladenosine). In a thirtieth embodiment, the *in vitro* synthesized RNA contains one or more nucleotides selected from Ψ (pseudouridine), m⁵C (5-methylcytidine) or both.

In a thirty-first embodiment, the surface coated substrate comprises magnetic beads.

In a thirty-second embodiment, the reagent on the surface coated substrate that binds to the nonradiolabeled tagged probe is: (a) streptavidin, wherein the nonradiolabeled tag is biotin; (b) avidin, wherein the nonradiolabeled tag is biotin; (c) an anti-biotin antibody, wherein the nonradiolabeled tag is biotin; (d) an anti-digoxigenin antibody, wherein the nonradiolabeled tag is digoxigenin; and (e) an anti-peptide antibody, wherein the nonradiolabeled tag is a peptide.

In a thirty-third embodiment, the uncapped triphosphate mRNA is detectable over a tested range of 0.1 to 90% with a linear response. In a thirty-fourth embodiment, the uncapped triphosphate mRNA is detectable over a tested range of 0.5 to 25% with a linear response.

In a thirty-fifth embodiment, the invention provides a radiolabel free method for detecting a capping reaction impurity in an RNA preparation, comprising the steps of: (a) hybridizing a nonradiolabeled tagged probe with the target RNA, wherein the nonradiolabeled tagged probe is complementary to the 5' end of the target RNA; (b) treating the hybridized RNA with RNAse H to cleave the 5' end of the RNA; (c) isolating the cleaved 5' end sequence, using magnetic beads coated with a reagent that binds to the nonradiolabeled tagged probe; (e) analyzing the cleaved 5' end sequence by LC-MS, and (f) detecting a capping reaction impurity in an RNA preparation. In a thirty-sixth embodiment, the method further includes the step of: (f) identifying a capping reaction impurity in an RNA preparation.

The method of the invention showed good sensitivity for detecting capping reaction impurities. Uncapped triphosphate mRNA spiked into 100 picomoles capped mRNA could be detected over the tested range of 0.5 to 25% with a linear response. The capping efficiency of several *Vaccinia* capped mRNA preparations was determined to be between 88 and 98% depending on the modification type and length of the mRNA.

In a thirty-seventh embodiment, the target RNA is synthesized *in vitro.* In a more specific embodiment, the *in vitro* synthesized RNA comprises a nucleotide selected from the group consisting of Ψ (pseudouridine); m⁵C (5-methylcytidine); m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms² m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f⁵C (5-formylcytidine); m⁵ Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m² ₂G (N²,N²-dimethylguanosine); m²Gm (N²,2'-O-dimethylguanosine); m²₂Gm (N²,N²2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine); cmnm⁵s²U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N⁶,2'-O-dimethyladenosine); m⁶ ₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); andac⁶A (N⁶-acetyladenosine). In a thirty-eighth, specific embodiment, the *in vitro* synthesized RNA comprises Ψ (pseudouridine), m⁵C (5-methylcytidine), or both nucleosides. In a thirty-ninth embodiment, the substrate of the surface coated substrate is magnetic beads.

In a fortieth embodiment, the reagent on the surface coated substrate that binds to the nonradiolabeled tagged probe is: (a) streptavidin, wherein the nonradiolabeled tag is biotin; (b) avidin, wherein the nonradiolabeled tag is biotin; (c) an anti-biotin antibody, wherein the nonradiolabeled tag is biotin; (d) an anti-digoxigenin antibody, wherein the nonradiolabeled tag is digoxigenin; or (e) an anti-peptide antibody, wherein the nonradiolabeled tag is a peptide.

### BRIEF DESCRIPTION OF THE DRAWINGS

FIG. 1 shows the enzymatic pathway involved in capping mRNA resulting in Cap0 and Cap1 (adopted from Ghosh & Lima, Wiley Interdiscip. Rev. RNA 1(1): 152-172 (2010)).
FIG. 2 is a schematic of the procedure for RNAse H cleavage of the mRNA and isolation of the cleavage fragment by magnetic beads. The arrows indicate the two cleavage sites observed by LC-MS.
FIG. 3 shows the workflow of cap orientation analysis showing the reaction products from the RppH enzyme treatment added to the RNAse H cleavage sequence. The reverse cap orientation p7mG... has a different mass than the monophosphate.

### DETAILED DESCRIPTION OF THE INVENTION

### Definitions

So that the invention can be more readily understood, some terms are here defined. Additional definitions may be set forth throughout the specification.

"3'" when used in a nucleotide position refers to a region or position in a polynucleotide or oligonucleotide 3' (*i.e.,* downstream) from another region or position in the same polynucleotide or oligonucleotide. The terms "3' end" and "3' terminus", as used herein in reference to a nucleic acid molecule, refer to the end of the nucleic acid which contains a free hydroxyl group attached to the 3' carbon of the terminal pentose sugar.

"5'" when used in a nucleotide position refers to a region or position in a polynucleotide or oligonucleotide 5' (*i.e*., upstream) from another region or position in the same polynucleotide or oligonucleotide. The term "5' end" and "5' terminus", as used herein in reference to a nucleic acid molecule, refers to the end of the nucleic acid molecule which contains a free hydroxyl or phosphate group attached to the 5' carbon of the terminal pentose sugar. In some embodiments of the invention, oligonucleotide primers comprise tracts of poly-adenosine at their 5' termini.

"Affinity", as used in the art, is a measure of the tightness with which a particular ligand binds to (*e.g*., associates non-covalently with) and/or the rate or frequency with which it dissociates from, its partner. The skilled artisan will know that several methods have been and can be used to determine affinity. Affinity is a measure of specific binding.

"Anneal," "hybridization", "hybridize" and grammatical equivalents thereof mean the formation of complexes (also called "duplexes" or "hybrids") between nucleotide sequences that are sufficiently complementary to form complexes by Watson-Crick base pairing or non-canonical base pairing. Annealing or hybridizing sequences need not have perfect complementary to provide stable hybrids. Stable hybrids can usually form where fewer than about 10% of the bases are mismatches. Accordingly, as used herein, the term "complementary" refers to a nucleic acid molecule that forms a stable duplex with its complement under particular conditions, generally where there is about 90%> or greater homology (*e.g*., about 95% or greater, about 98%> or greater, or about 99% or greater homology). The skilled artisan understands how to estimate and adjust the stringency of hybridization conditions such that sequences that have at least a desired level of complementarity will stably hybridize, while those having lower complementarity will not. For examples of hybridization conditions, see Green & Sambrook, Molecular Cloning: A Laboratory Manual, Fourth Edition (Cold Spring Harbor Press, Plainview, NY, 2012) and Ausubel, Current Protocols in Molecular Biology (John Wiley & Sons, Somerset NJ USA, 2016). Complementarity between two nucleic acid molecules and duplex formation is "complete", "total" or "perfect" if all the nucleic acid's bases are matched, and is otherwise "partial".

"ARCA" are anti-reverse" cap analogues, which are commercially available from TriLink, San Diego CA 92121 USA. In cells, the ribosome translates mRNA into proteins. mRNA in eukaryotic cells have a 5' cap [m⁷G(5')ppp(5')G]. A different cap can be incorporated in the mRNA transcription (*e.g.,* by *in vitro* transcription) by including a mixture of cap analog and GTP. Thus, approximately 80% of synthesized mRNA will possess a 5' cap, while the remaining 20% will have a 5' triphosphate. The first cap analog to be introduced was m⁷G(5')ppp(5')G. 50% of the time, this cap is inserted in the correct orientation to enhance translation. The other 50% of molecules are not substrates for efficient translation, reducing the specific activity of the RNA transcript. More recently, Anti-Reverse Cap Analog (ARCA) (Trilink, part # N-7003) was introduced [3' O-Me-m⁷G(5')ppp(5')G] Stepinski J et al., RNA 7(10):1486-95 (October 2001). ARCA can only insert in the proper orientation, resulting in capped mRNAs that are translated twice as efficiently as those initiated with m⁷G(5')ppp(5')G. Efficient translation of the mRNA into protein also requires a poly(A) tail. This can be introduced by including a poly(dT) stretch at the end of the transcription template. Often this is accomplished by a PCR step that utilizes a primer containing the poly(dT) stretch.

A "biotin tag" is a biotin chemically attached to a compound of interest. Thus, a "biotin tagged probe" is a polynucleotide probe that has a biotin tag covalently attached to the probe.

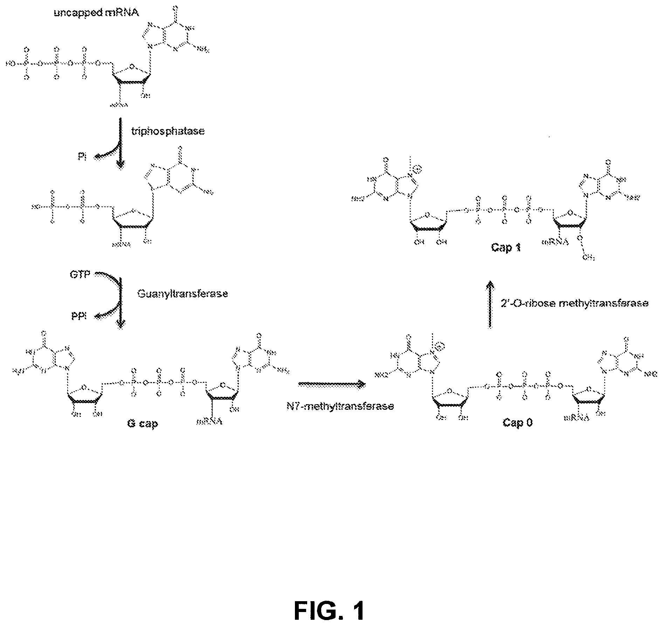

"Cap0" is a m7GpppG cap. See, FIG. 1 and SEQ ID NO.: 1. 5' terminal caps are commercially available, *e.g.,* from TriLink BioTechnologies, Inc., San Diego CA USA.

"Cap1" is a m7GpppmG cap, wherein Cap0 further methylated on the 2' OH of the penultimate guanine. See, FIG. 1 and SEQ ID NO.: 6. 5' terminal caps are commercially available, *e.g.,* from TriLink BioTechnologies, Inc., San Diego CA USA.

"Chromatography" is a technique for separation of mixtures. The mixture is typically dissolved in a fluid called the "mobile phase," which carries it through a structure holding another material called the "stationary phase." Examples include LC and HPLC.

"Compound" is any naturally occurring or non-naturally occurring (*i.e.,* synthetic or recombinant) molecule, such as a biological macromolecule (*e.g*., nucleic acid, polypeptide or protein), organic or inorganic molecule, or an extract made from biological materials such as bacteria, plants, fungi, or animal (particularly mammalian, including human) cells or tissues. The compound may be a single molecule or a mixture or complex of at least two molecules.

"Control" has the art-understood meaning of being a standard against which results are compared. In some embodiments, a control is a reaction or assay that is performed simultaneously with a test reaction or assay to provide a comparator. In one experiment, the "test" (*i.e.,* the variable being tested) is applied. In the second experiment, the "control," the variable being tested is not applied. In some embodiments, a control is a historical control (*i.e.,* of a test or assay performed previously, or an amount or result that is previously known). In some embodiments, a control is or comprises a printed or otherwise saved record. A control may be a positive control or a negative control.

Homology" means the overall relatedness between nucleic acid molecules (*e.g.* DNA molecules or RNA molecules) or between polypeptide molecules. The term "homologous" necessarily refers to a comparison between at least two sequences (polynucleotide or polypeptide sequences).

"HPLC" is high performance liquid chromatography, previously known as high pressure liquid chromatography, a form of column chromatography that pumps a sample mixture or analyte in a solvent (known as the mobile phase) at high pressure through a column with chromatographic packing material (stationary phase). Many HPLC techniques are known in the biotechnological arts. For more information, see the HPLC Primer (2015) available from Waters Corporation, Milford MA USA.

"IVT" is *in vitro* transcription of ribonucleic acid (RNA) from a deoxyribonucleic acid (DNA) template. Many IVT techniques are known in the biotechnological arts. For information, see *The Basics: In Vitro Transcription* (2015), available from Thermo Fisher Scientific Inc., Waltham MA USA.

"LC" is liquid chromatography, a technique used to separate a sample into its individual parts. This separation occurs based on the interactions of the sample with the mobile and stationary phases. Many LC techniques are known in the biotechnological arts. For more information, see the Beginners Guide to UPLC (2015) and the HPLC Primer (2015), both available from Waters Corporation, Milford MA USA.

"Modified" means a changed state or structure of a molecule of the invention. A "modified" mRNA contains ribonucleosides that encompass modifications relative to the standard guanine (G), adenine (A), cytidine (C), and uridine (U) nucleosides. The nonstandard nucleosides can be naturally occurring or non-naturally occurring. RNA can be modified in many ways including chemically, structurally, and functionally, by methods known to those of skill in the biotechnological arts. Such RNA modifications can include, *e.g.,* modifications normally introduced post-transcriptionally to mammalian cell mRNA. Moreover, mRNA molecules can be modified by the introduction during transcription of natural and non-natural nucleosides or nucleotides, as described in U.S. Pat. No. 8,278,036 (Karikó et al.); U.S. Pat. Appl. No. 2013/0102034 (Schrum); U.S. Pat. Appl. No. 2013/0115272 (deFougerolles et al.) and U.S. Pat. Appl. No. 2013/0123481 (deFougerolles et al.).

"MS" is mass spectrometry, an analytical chemistry technique that helps identify the amount and type of chemicals present in a sample by measuring the mass-to-charge ratio and abundance of gas-phase ions. A mass spectrum (plural spectra) is a plot of the ion signal as a function of the mass-to-charge ratio. Many MS techniques are known in the biotechnological arts. For more information, see the MS Primer (2015) available from Waters Corporation, Milford MA USA. See also, Basiri et al., Bioanalysis 6(11): 1525-1542 (2014).

"mRNA" is messenger RNA, including eukaryotic messenger RNA. mRNAs convey genetic information from DNA to the ribosome, where they specify the amino acid sequence of the protein products of gene expression by a process known as transcription. Structurally and informationally, mRNA encodes the information for a protein in a coding region. Eukaryotic mRNA can begin at the 5' end with an mRNA cap that is enzymatically synthesized after the mRNA has been transcribed by an RNA polymerase *in vitro.* The mRNA cap facilitates translation initiation while avoiding recognition of the mRNA as foreign and protects the mRNA from 5' exonuclease mediated degradation. The 5' cap can be a modified guanine nucleotide that is linked to the 5' end of an RNA molecule using a 5'-5' triphosphate linkage. The 5' cap can also be a 5' cap analog, such as 5' diguanosine cap, tetraphosphate cap analogs having a methylene-bis(phosphonate) moiety (see *e.g.,* Rydzik AM et al., Org. Biomol. Chem. 7(22):4763-76) (2009), dinucleotide cap analogs having a phosphorothioate modification (see *e.g.,* Kowalska J et al., RNA 14(6):1119-1131 (2008)), cap analogs having a sulfur substitution for a non-bridging oxygen (see *e.g.,* Grudzien-Nogalska E et al., RNA 13(10): 1745-1755 (2007)), N7-benzylated dinucleoside tetraphosphate analogs (see *e.g.,* Grudzien E et al. RNA 10(9):1479-1487 (2004)), or anti-reverse cap analogs (see *e.g.,* Jemielity J et al., (2003) RNA 9(9): 1108-1122 and Stepinski J et al., RNA 7(10):1486-1495 (2001)). The 5' cap analog can be a 5' diguanosine cap. See also, methods for capping by New England Biolabs (Beverly MA USA).

A "nonradiolabeled tag" is one or more non-radioactive markers, signals, or moieties which are attached, incorporated or associated with another entity (such as a polynucleotide, such as RNA) that is readily detected by methods known in the art including fluorescence, chemiluminescence, enzymatic activity, absorbance and the like. Detectable labels include fluorophores, chromophores, enzymes, dyes, metal ions, ligands such as biotin, avidin, strepavidin, digoxigenin and haptens, quantum dots, and the like. Detectable labels may be located at any position in the RNAs disclosed herein. A "nonradiolabeled tagged probe" is a polynucleotide probe that has a covalently attached nonradiolabeled tag.

"Nucleoside" or "nucleobase" refer to a base (adenine (A), guanine (G), cytosine (C), uracil (U), thymine (T) and analogs thereof) linked to a carbohydrate, for example D-ribose (in RNA) or 2'-deoxy-D-ribose (in DNA), through an N-glycosidic bond between the anomeric carbon of the carbohydrate and the nucleobase. When the nucleobase is purine (*e.g.,* A or G), the ribose sugar is generally attached to the N9-position of the heterocyclic ring of the purine. When the nucleobase is pyrimidine (*e.g.,* C, T or U), the sugar is usually attached to the N1 -position of the heterocyclic ring. The carbohydrate may be substituted or unsubstituted. Substituted ribose sugars include, but are not limited to, those in which one or more of the carbon atoms, for example the 2'-carbon atom, is substituted with one or more of the same or different -Cl, -F, -R, -OR, -NR₂ or halogen groups, where each R is independently H, C₁-C₆ alkyl or C₅-C₁₄ aryl. Ribose examples include ribose, 2'-deoxyribose, 2',3'-dideoxyribose, 2'-haloribose, 2'-fluororibose, 2'-chlororibose, and 2'-alkylribose, *e.g.,* 2'-O-methyl, 4'-alpha-anomeric nucleotides, 2*-4*- and 3*-4*-linked and other "locked" or "LNA," bicyclic sugar modifications. See, WO 98/22489 (Takeshi Imanishi); WO 98/39352 (Exiqon A/S, Santaris Pharma A/S); and WO 99/14226 (Exiqon A/S).

"Nucleotide" is a nucleoside in a phosphorylated form (a phosphate ester of a nucleoside), as a monomer unit or within a polynucleotide polymer. A "nucleotide 5'-triphosphate" is a nucleotide with a triphosphate ester group at the 5' position, sometimes denoted as "NTP", or "dNTP" and "ddNTP" to particularly point out the structural features of the ribose sugar. The triphosphate ester group may include sulfur substitutions for the various oxygen moieties, *e.g.,* α-thio-nucleotide 5'- triphosphates. Nucleotides can exist in the mono-, di-, or tri-phosphorylated forms. The carbon atoms of the ribose present in nucleotides are designated with a prime character (') to distinguish them from the backbone numbering in the bases. For a review of polynucleotide and nucleic acid chemistry see Shabarova Z & Bogdanov A, Advanced Organic Chemistry of Nucleic Acids (VCH, New York, 1994).

"Nucleic acid", "nucleic acid molecule", "polynucleotide" and "oligonucleotide" refer interchangeably to polymers of nucleotide monomers or analogs thereof, such as deoxyribonucleic acid (DNA) and ribonucleic acid (RNA) and combinations thereof. The nucleotides may be genomic, synthetic or semi-synthetic in origin. Unless otherwise stated, the terms encompass nucleic acid-like structures with synthetic backbones, as well as amplification products. The length of these polymers (*i.e.,* the number of nucleotides it contains) can vary widely, often depending on their intended function or use. Polynucleotides can be linear, branched linear, or circular molecules. Polynucleotides also have associated counter ions, such as H⁺, NH₄⁺, trialkylammonium, Mg⁺, Na⁺ and the like. A polynucleotide may be composed entirely of deoxyribonucleotides, entirely of ribonucleotides, or chimeric mixtures thereof. Polynucleotides may be composed of internucleotide nucleobase and sugar analogs.

An "oligonucleotide" is a polynucleotide that comprises between about 5 and about 150 nucleotides, *e.g.,* between about 10 and about 100 nucleotides, between about 15 and about 75 nucleotides, or between about 15 and about 50 nucleotides. Throughout the specification, whenever an oligonucleotide is represented by a sequence of letters (chosen, for example, from the four base letters: A (adenosine), C (cytidine), G (guanosine), and T (thymidine), the nucleotides are presented in the 5' to 3' order from the left to the right. A "polynucleotide sequence" refers to the sequence of nucleotide monomers along the polymer. Unless denoted otherwise, whenever a polynucleotide sequence is represented, the nucleotides are in 5' to 3' orientation from left to right. Nucleic acids, polynucleotides and oligonucleotides may be comprised of standard nucleotide bases or substituted with nucleotide isoform analogs, including, but not limited to iso-C and iso-G bases, which may hybridize more or less permissibly than standard bases, and which will preferentially hybridize with complementary isoform analog bases. Many such isoform bases are described, *e.g.,* by Benner et al., Cold Spring Harb. Symp. Quant. Biol. 52: 53-63 (1987). Analogs of naturally occurring nucleotide monomers include, for example, 7-deazaadenine, 7-deazaguanine, 7-deaza-8-azaguanine, 7-deaza-8-azaadenine, 7-methylguanine, inosine, nebularine, nitropyrrole, nitroindole, 2-aminopurine, 2-amino-6-chloropurine, 2,6-diaminopurine, hypoxanthine, pseudouridine (Ψ), pseudocytosine. pseudoisocytosine, 5-propynylcytosine, isocytosine, isoguanine), 7-deazaguanine, 2-azapurine, 2-thiopyrimidine, 6-thioguanine, 4-thiothymine, 4-thiouracil, O-6-methylguanine, N6-methyladenine, O-4-methylthymine, 5,6-dihydrothymine, 5,6-dihydrouracil, 4-methylindole, pyrazolo[3,4-D]pyrimidines, "PPG" (U.S. Pat. Nos. 6,143,877 (Meyer)) and ethenoadenine (Fasman, in Practical Handbook of Biochemistry and Molecular Biology, pp. 385-394 (CRC Press, Boca Raton FL USA, 1989)). See also, the nucleosides described in U.S. Pat. No. 8,278,036 (Karikó et al.); WO 2015/095351 (Novartis AG); Karikó K et al. Curr. Opin. Drug Disc. Devel. 10(5): 523-532 (2007); Karikó K et al. Mol. Therap. 16(11): 1833-1840 (2008) and Anderson BR et al., Nucleic Acids Res. 38(17): 5884-5892 (2010).

"PAGE" is polyacrylamide gel electrophoresis, a technique widely used in the biotechnological arts to separate biological macromolecules, *e.g.,* nucleic acids, according to their electrophoretic mobility. Many PAGE techniques are known in the biotechnological arts. For more information, see Green & Sambrook, Molecular Cloning: A Laboratory Manual, Fourth Edition (Cold Spring Harbor Press, Plainview, NY, 2012).

"Primers" are short nucleic acid sequences. Polymerase chain reaction (PCR) primers are typically oligonucleotides of short length (*e.g*., 8-30 nucleotides) that are used in polymerase chain reactions. PCR primers and hybridization probes can readily be developed and produced by those of skill in the art, using sequence information from the target sequence. See, Green & Sambrook, Molecular Cloning: A Laboratory Manual, Fourth Edition (Cold Spring Harbor Press, Plainview, NY, 2012).

A "probe" as used herein is an oligonucleotide probe, a nucleic acid molecule which typically ranges in size from about 50-100 nucleotides to several hundred nucleotides to several thousand nucleotides in length, in whole number increments. A probe can be any suitable length for use in the method of the invention described herein. Such a molecule is typically used to identify a specific nucleic acid sequence in a sample by hybridizing to the specific nucleic acid sequence under stringent hybridization conditions. Hybridization conditions are known in the biotechnological arts. See, *e.g.,* Green & Sambrook, Molecular Cloning: A Laboratory Manual, Fourth Edition (Cold Spring Harbor Press, Plainview, NY, 2012).

A "reagent that binds to a nonradiolabeled tagged probe" or a "reagent that binds to a nonradiolabeled tag" is a reagent that binds to a nonradiolabeled tag that can be attached to the oligonucleotide probe used in the method of the invention. Examples of a reagent that binds to a nonradiolabeled tagged probe include (a) streptavidin, where the nonradiolabeled tag is biotin; (b) avidin, where the nonradiolabeled tag is biotin; (c) an anti-biotin antibody, where the nonradiolabeled tag is biotin; (d) an anti-digoxigenin antibody, where the nonradiolabeled tag is digoxigenin; and (e) an anti-peptide antibody, wherein the nonradiolabeled tag is a peptide. Other pairs of nonradiolabeled tags and reagents that bind to the nonradiolabeled tagged probe are known to those of skill in the biotechnological arts. See, *e.g.,* Green & Sambrook, Molecular Cloning: A Laboratory Manual, Fourth Edition (Cold Spring Harbor Press, Plainview, NY, 2012).

"RNA" is ribonucleic acid, a ribonuceloside polymer. Each nucleotide in an RNA molecule contains a ribose sugar, with carbons numbered 1' through 5'. A base is attached to the 1' position. In general, the bases are adenine (A), cytosine (C), guanine (G), or uracil (U), although many modifications are known to those of skill in the art. For example, as described herein, an RNA may contain one or more pseudouracil (Ψ) base, such that the pseudouridine nucleotides are substituted for uridine nucleotides. Many other RNA modifications are known to those of skill in the art, as described herein. Procedures for isolating and producing RNA are known to the skilled artisan, such as a laboratory scientist. See, Green & Sambrook, Molecular Cloning: A Laboratory Manual, Fourth Edition (Cold Spring Harbor Press, Plainview NY, 2012).

"RNase H" is ribonuclease H, a family of non-sequence-specific endonucleases that catalyze the cleavage of RNA by a hydrolytic mechanism. RNase H's ribonuclease activity cleaves the 3' O-P bond of RNA in a DNA/RNA duplex substrate to produce 3'-hydroxyl and 5'-phosphate terminated products.

"RppH" is the 5' RNA pyrophosphohydrolase enzyme.

"snRNA" is small nuclear RNA. snRNAs contain unique 5'-caps. Sm-class snRNAs are found with 5'-trimethylguanosine caps, while Lsm-class snRNAs are found with 5'-monomethylphosphate caps. See, Matera, A et al., Nature Rev. Mol. Cell Biol. 8(3): 209-220 (March 2007).

A "substitution" is a mutation that exchanges one base for another (*i.e.,* a change in a single "chemical letter" such as switching an A to a G). Such a substitution could (a) change a codon to one that encodes a different amino acid and cause a small change in the protein produced; (b) change a codon to one that encodes the same amino acid and causes no change in the protein produced ("silent mutations"); or (c) change an amino-acid-coding codon to a single "stop" codon and cause an incomplete protein.

A "surface coated substrate" is a substrate that is coated with a reagent that binds to a nonradiolabeled tagged probe. In one embodiment, the substrate of the surface coated substrate is magnetic beads.

"Synthetic" means produced, prepared, or manufactured by the human intervention. Synthesis of polynucleotides or polypeptides or other molecules of the invention may be chemical or enzymatic.

The term "target" refers to a molecule of interest. "Target RNA" is an RNA of interest, which can be analyzed by the method of the invention.

### Sequence identification numbers

| TABLE 1 | | |
|---|---|---|
| Nucleotide Sequences | | |
| Polynucleotide (with 5' cap) | Sequence | SEQ ID NO. |
| Cap0 | m7GpppGGGAGACGCGUGUUAAAUAACA | 1 |
| uncapped triphosphate | pppGGGAGACGCGUGUUAAAUAACA | 2 |
| uncapped diphosphate | ppGGGAGACGCGUGUUAAAUAACA | 3 |
| uncapped monophosphate (the expected peak for the RppH enzymatic product with the correct orientation) | pGGGAGACGCGUGUUAAAUAACA (gggagacgcguguuaaauaaca) | 4 |
| Rev cap | pm7GGGAGACGCGUGUUAAAUAACA | 5 |
| Cap1 | m7GpppmGGGAGACGCGUGUUAAAUAACA | 6 |
| Cap1 triphosphate | pppmGGGAGACGCGUGUUAAAUAACA | 7 |
| Cap1 diphosphate | ppmGGGAGACGCGUGUUAAAUAACA | 8 |
| Cap1 monophosphate | pmGGGAGACGCGUGUUAAAUAACA | 9 |
| G cap | GpppGGGAGACGCGUGUUAAAUAACA | 10 |
| 5mC Cap0 | m7GpppGGGAGAmCGmCGUGUUAAAUAAmCA | 11 |
| 5mC uncapped triphosphate | pppGGGAGAmCGmCGUGUUAAAUAAmCA | 12 |
| 5mC uncapped diphosphate | ppGGGAGAmCGmCGUGUUAAAUAAmCA | 13 |
| 5mC G cap | GpppGGGAGAmCGmCGUGUUAAAUAAmCA | 14 |
| Cleavage probe | TTTGTTmAmUmUmUmAmAmCmAmCmGmCmGmUmCmUmC mCmC/3BioTEG/ | 15 |
| "48mer" mRNA substrate | rGrGrGrArGrArCrGrCrGrUrGrUrUrArArArUrA rArCrArArArUrCrUrCrArArCrArCrArArCrArU rArUrArCrArArArArCrA (gggagacgcguguuaaauaacaaaucucaacacaaca uauacaaaaca) | 16 |
| *Photinus pyralis* (firefly) luciferase mRNA | cugcagaaauaacuagguacuaagcccguuugugaaaa guggccaaacccauaaauuggcaauuacaauaaagaa gcuaaaauuguggucaaacucacaaacauuuuauuau auacauuuuaguagcugaugcuuauaaaagcaauauuu aaaucguaaacaacaaauaaaauaaaauuuaaacgaug ugauuaagagccaaagguccucuagaaaagguauuua agcaacggaauuccuuuguguuacauucuugaaugucg cucgcagugacauuagcauuccgguacuguugguaaaa uggaagacgccaaaaacauaaagaaaggcccggcgcca uucuauccucuagaggauggaaccgcuggagagcaacu gcauaaggcuaugaagagauacgcccugguuccuggaa caauugcuuuugugaguauuucugucugauuucuuucg aguuaacgaaauguucuuauguuucuuuagacagaugc acauaucgaggugaacaucacguacgcggaauacuucg aaauguccguucgguuggcagaagcuaugaaacgauau gggcugaauacaaaucacagaaucgucguaugcaguga aaacucucuucaauucuuuaugccgguguugggcgcgu uauuuaucggaguugcaguugcgcccgcgaacgacauu uauaaugaacguaagcacccucgccaucagaccaaagg | 17 |
| | gaaugacguauuuaauuuuuaaggugaauugcucaaca guaugaacauuucgcagccuaccguaguguuuguuucc aaaaaggguugcaaaaaauuuugaacgugcaaaaaa auuaccaauaauccagaaaauuauuaucauggauucua aaacggauuaccagggauuucagucgauguacacguuc gucacaucucaucuaccucccgguuuuaaugaauacga uuuuguaccagaguccuuugaucgugacaaaacaauug cacugauaaugaauuccucuggaucuacugguuaccu aagggguguggcccuuccgcauagaacugccugcgucag auucucgcaugccagguaugucguauaacaagagauua aguaauguugcuacacacauuguagagauccuauuuuu ggcaaucaaaucauuccggauacugcgauuuuaagugu uguuccauuccaucacgguuuggaauguuuacuacac ucggauauuugauauguggauuucgagucgucuuaaug uauagauuugaagaagagcuguuuuuacgaucccuuca ggauuacaaaauucaaagugcguugcuaguaccaaccc uauuuucauucuucgccaaaagcacucugauugacaaa uacgauuuaucuaauuuacacgaaauugcuucuggggg cgcaccucuuucgaaagaagucggggaagcgguugcaa aacggugaguuaagcgcauugcuaguauuucaaggcuc uaaaacggcgcguagcuuccaucuuccaggauacgac aaggauaugggcucacugagacuacaucagcuauucug auuacacccgagggggaugauaaaccgggcgcggucgg uaaaguuguuccauuuuuugaagcgaagguuguggauc uggauaccgggaaaacgcugggcguuaaucagagaggc gaauuaugugucagaggaccuaugauuauguccgguua uguaaacaauccggaagcgaccaacgccuugauugaca aggauggauggcuacauucuggagacauagcuuacugg gacgaagacgaacacuucuucauaguugaccgcuugaa gucuuuaauuaaauacaaaggauaucagguaaugaaga uuuuuacaugcacacacgcuacaauaccuguaggugggc ccccgcugaauuggaaucgauauuguuacaacacccca acaucuucgacgcgggcguggcaggucuucccgacgau gacgccggugaacuucccgccgccguuguuguuuugga gcacggaaagacgaugacggaaaaagagaucguggauu acgucgccaguaaaugaauucguuuuacguuacucgua cuacaauucuuuucauaggucaaguaacaaccgcgaaa aaguugcgcggaggaguuguguuuguggacgaaguacc gaaaggucuuaccggaaaacucgacgcaagaaaaauca gagagauccucauaaaggccaagaagggcggaaagucc aaauuguaaaauguaacuguauucagcgaugacgaaau ucuuagcuauuguaauauuauaugcaaauugaugaaug guaauuuuguaauugugggucacuguacuauuuuaacg aauaauaaaaucagguauagguaacuaaaaa | |

### 5'-end capping

Eukaryotic cellular mRNA is modified on the 5' end by the incorporation of a N7-methylguanosine triphosphate cap (m7GpppG....mRNA). Thus, N7-methylguanosine is the final base present on the 5' end of mRNA. Muthukrishnan S et al., Nature 255 (5503): 33-37 (1975); Shatkin AJ et al., Cell 9(4 PT 2): 645-653 (1976). This 5' dinucleotide cap is an important transcriptional regulatory element, and its role in eukaryotic mRNA translation has been widely studied. mRNA without the m7GpppG cap are not recognized by the initiation factor protein complex elF4E and are not exported from the nucleus and translated. Topisirovic I et al., Wiley Interdiscip Rev. RNA 2(2): 277-298 (2011). The 5' dinucleotide cap influences the lifetime of mRNA by protecting the 5' end of the mRNA from exonuclease degradation. Green MR et al., Cell 32 (3):681-694 (1983); Furuichi Y et al., Nature 266(5599): 235-239 (1997); Coutts M et al., Biochim. Biophys. Acta 1173(1): 49-56 (1993). mRNA that is produced by *in vitro* transcription (IVT) from a DNA template must be capped to be translated.

There were two frequently used ways of capping mRNA produced by IVT. In the first way, a cocktail of capping enzymes (usually from *Vaccinia* virus) can be added to the synthesized mRNA. This mixture will add guanine from GTP to form the terminal 5'-5' GpppG cap which is then methylated to produce the m⁷GpppG cap (CapO) or further methylated on the 2' OH of the penultimate guanine to produce m⁷GpppmG (Cap1) See, FIG. 1. This approach is known to as "post-transcriptional" capping. Tcherepanova IY et al., BMC Mol. Biol. 9: 90 (2008).

In the second way, the mRNA is capped "co-transcriptionally" as it is transcribed, by adding the dinucleotide cap analogue m⁷GpppG to the IVT reaction. The RNA polymerase incorporates the m7GpppG into the 5' terminal of the mRNA in place of GTP. mRNA produced this way is not 100% capped due to the competition between GTP and the dinucleotide cap analogue during synthesis. Dinucleotide cap analogues can be incorporated in the reverse orientation (GpppGm⁷) so that the m⁷G is no longer the terminal base. Up to 50% of mRNA capped "co-transcriptionally" can contain reverse caps. Pasquinelli AE et al., RNA 1(9): 957-967 (1995).

Because cap orientation is critical to translation of mRNA, "anti-reverse" cap analogues (ARCA) were developed to ensure that caps are incorporated in the correct orientation. Peng ZH et al., Org. Lett. 4 (2):161-164 (2002); Stepinski J et al., RNA 7(10): 1486-1495 (2001). With ARCA dinucleotides, the 3' ribose -OH on the N7-methylguanosine is methylated in ARCA dinucleotides to prevent capping from that side of the dinucleotide. Although the ARCA capping method ensures correct orientation of the 5' cap, "co-transcriptionally" produced mRNA inherently produces mRNA with a triphosphate on the 5' end, due to the competition between GTP and the cap motif during transcription. The 5' triphosphate is immunogenic. Its presence must be mitigated for the mRNA not to be cytotoxic. Hornung V et al., Science 314(5801): 994-997 (2006). Due to the importance of the 5' cap, knowing the extent of capping and the orientation of that cap is vital to understanding the efficiency of mRNA translation.

### A radiolabel free method for identifying the 5' end cap on a target RNA

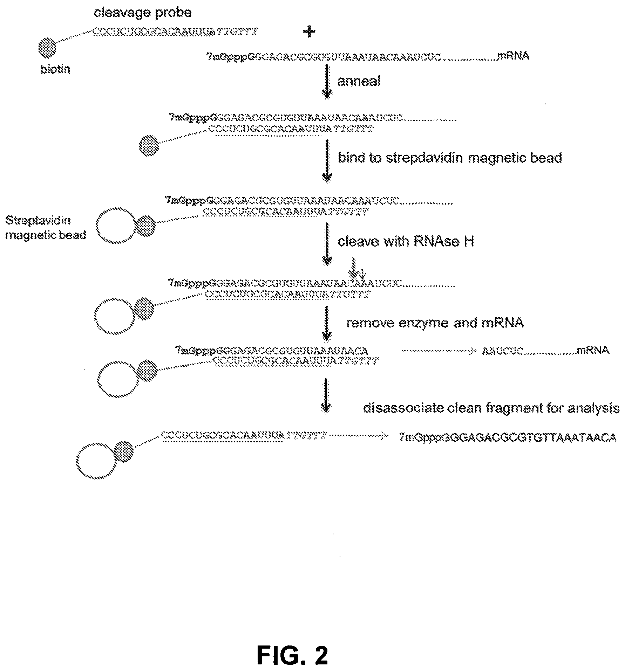

Full-length mRNA is much too large to be analyzed directly by LC-MS, so the method of the invention first uses a technique first described by Lapham & Crothers, RNA 2(3): 289-296 (1996), to cleave short sequences from the 5' end of the mRNA at a specific location. This technique uses a biotin tagged RNAse H cleavage probe that is complementary to the 5' end of the mRNA (see, *e.g.* SEQ ID NO.: 15, which is complementary to the 5' end of a 2.2K mRNA and 9K mRNA, both described below). FIG. 2 presents a schematic of this workflow.

Materials and methods In this forty-first embodiment, the cleavage probe has an RNA sequence that contains 2' O-methyl modifications except at the 3' end cleavage site, which contains 4-6 DNA nucleotides (see, *e.g.* SEQ ID NO.: 15). The terminal stretch of DNA directs the RNAse H to cleave the mRNA at a specific location, because the 2' O-methyl modified RNA is not recognized by RNAse H as a cleavage site. Inoue H et al., FEBS Lett. 215(2): 327-330 (1987).

In one example, we tested the method of the invention using two target RNA, which were two different length mRNAs, one roughly 2200 nucleotides (*Photinus pyralis* (firefly) luciferase mRNA, SEQ ID NO.: 17) and another roughly 9000 nucleotides. The first 50 nucleotides on the 5' end of each mRNA were the same sequence, so we used one RNAse H cleavage probe for both. The mRNA was prepared using standard in vitro transcription (IVT) procedures and then capped using the *Vaccinia* Capping System from New England Biolabs, Ipswich MA USA (Part# M2080S). Following capping, the mRNA was precipitated with LiCI and then taken up in water.

For mRNA containing Ψ (pseudouridine) or m⁵C (5-methylcytidine), the corresponding modified NTPs were used instead of U (uridine) or C (cytidine) in the regular mRNA synthesis reactions.

For examples of incorporation of Ψ (pseudouridine) or m⁵C (5-methylcytidine) into mRNA, see, U.S. Pat. No. 8,278,036 (Karikó et al.); WO 2015/095351 (Novartis AG); Karikó K et al. Curr. Opin. Drug Disc. Devel. 10(5): 523-532 (2007); Karikó K et al. Mol. Therap. 16(11): 1833-1840 (2008) and Anderson BR et al., Nucleic Acids Res. 38(17): 5884-5892 (2010). U.S. Pat. No. 8,278,036 (Karikó et al.) also describes the incorporation of other nucleotides into mRNA, including m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms² m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f³C (5-formylcytidine); m⁵ Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m² ₂G (N²,N²-dimethylguanosine); m²Gm (N²,2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine); cmnm⁵s²U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N⁶,2'-O-dimethyladenosine); m⁶ ₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); or ac⁶A (N⁶-acetyladenosine). Each possibility represents a separate embodiment of the method of the invention.

The length and integrity of both the 2K mRNA the 9K mRNA was confirmed by gel electrophoresis on a BioRad Experion RNA analyzer (Hercules CA USA).

Annealing mRNA and cleavage probe: Annealing the cleavage probe and mRNA was done in 100-120 µL of the RNAse H reaction buffer. A typical mixture consisted of 500 pmol RNAse H probe (5 µL), 10X RNase H reaction buffer, (12 µL), and 100 pmol mRNA (103 µL) to ensure that the RNAse H reaction buffer was at the recommended 1X concentration. The RNAse H cleavage probe was ordered from Integrated DNA Technologies (Coralville IA USA) and used directly. The cleavage probe had the following sequence, 5' to 3':
TTTGTTmAmUmUmUmAmAmCmAmCmGmCmGmUmCmUmCmCmC/3BioTEG/ (SEQ ID NO.: 15). Bases with a preceding m are 2' O-methylribose modified RNA; bases with no lower case prefix are DNA.

To ensure that all mRNA was bound to the cleavage probe, a 5:1 excess of probe was used. The 500 pmol is the maximum amount of biotin tagged oligo that can be bound to the bead according to the manufacturer and our own tests have supported this number. The mixture of cleavage probe and mRNA was annealed in a thermocycler at 95°C for 5 minutes, then ramped down at 2 minute intervals to 65°C, 55°C, 40°C and finally to 22°C.

Binding to beads and RNAse H cleavage: Streptavidin coated magnetic beads (Dynabeads MyOne Streptavidin C1, 10mg/mL part # 65002) were obtained from Invitrogen (Life Technologies, Grand Island NY USA) and prepared for use according to manufacturer protocols. Briefly, using a magnet to condense the beads, the storage solution was removed and the beads were mixed with an equal volume of 0.1 M NaOH + 0.05 M NaCl. This solution was removed and the beads were then resuspended in an equal volume of 0.1M NaCl and kept until needed.

In one example, immobilization of the cleavage probe was done with 100 µL of the 10mg/ml beads that had been prepared according to Invitrogen procedure. Prior to use, the 0.1M NaCl storage buffer was removed from the beads by magnet or centrifugation at 15000xg for 5 minutes. The annealed mRNA and probe solution (120 µL) was added to the streptavidin beads and incubated for 30 minutes at room temperature with gentle rotation of the tube to ensure all biotin labeled probe was bound.

Next, 10 µL of RNAse H (50 units) was added, mixed with a pipette, and incubated for 3 hours at 37°C. RNAse H and RNase H reaction buffer were obtained from New England Biolabs, Ipswich MA USA (Part # M0297L).

After the 5' end of the mRNA is cleaved, the nonradiolabeled tagged cleavage probe (see, *e.g.* SEQ ID NO.: 15, which is a biotin tagged probe) is still bound to the cleaved fragment as a duplex. This duplex containing the 5' fragment is captured on streptavidin magnetic beads, to isolate the cleaved fragment from the remaining mRNA and reaction buffer.

After three hours, the beads were magnetically condensed and the supernatant was removed and discarded without touching the beads. The beads were then washed with 100 µL of washing buffer (5 mM Tris-HCl, (Ph7.5), 0.5 mM EDTA, 1M NaCl) by pipetting up and down. After mixing, samples were placed on a magnet for two minutes and the supernatant removed. This washing step was repeated three times with washing buffer to remove enzyme, mRNA, and reaction buffer and then followed by three washes with distilled water to remove excess salt.

Once cleaned of the reaction components, the duplex containing the 5' fragment is released from the beads and analyzed with LC-MS. The coupling of the RNase H cleavage probe to a magnetic bead provides an easy platform for isolating and cleaning the cleavage product from buffers not compatible with LC-MS.

Elution: The distilled water was removed from the beads and the bound mRNA was released from the probe by adding 100 µL of 75% MeOH that had been heated to 80°C. This mixture was heated to 80°C on a hot plate for 3 minutes, then placed on a magnet for 3 minutes. The supernatant containing the cleavage product and released probe was then collected and dried down to 10 µL with an evaporative centrifuge at RT for ∼45 min. The sample was then re-suspended in 50 µL of 100 µM EDTA/1% MeOH for LC-MS analysis.

LC-MS Analysis: Analysis of the cleaved 5' mRNA fragment was conducted with an Acuity UPLC (Waters, Milford MA USA) connected to a QExactive orbitrap (Thermo Scientific, Grand Island NY USA). Mobile phase A consisted of 200 mM hexafluoroisopropanol + 8.15 mM triethylamine, pH7.9 and mobile phase B was 100% methanol. A Waters Acuity C18, 2.1X50mm column heated to 75°C with a flowrate of 300 µl/min was used for all analyses. The gradient profile for elution started at 5% B for 1 minute followed by a linear ramp to 25% over 12 minutes. At twelve minutes, a one-minute rinse at 90% B began, followed by a return to 5% B at 13 minutes.

Mass spectra were obtained in the negative ion mode, over a scan range of 600-3000 *m*/*z* at either 70,000 or 140,000 resolution. Source and capillary temperatures were set to 400°C. Spectra were analyzed using Promass Software (Novatia, Newtown PA USA) in the high-resolution mode.

Results for identification of cleavage products: The unique mass of cleavage fragments having an m7GpppG cap compared to those that contain a diphosphate, triphosphate, or unmethylated G cap (GpppG) enables identification of these fragments and an estimate of capping efficiency, as shown in TABLE 2.

**TABLE 2**

| Observed and theoretical cleavage ions for various mRNA cleavage products | | | | |
|---|---|---|---|---|
| | Probe Cleavage products 5' to 3' | Mono iso mol wt. | -3 Charge state* | observed |
| Cap0 | m7GpppGGGAGACG CGUGUUAAAU AACA | 7645.0256 | 2546.9980 | 2546.9993 |
| uncapped triphos | pppGGGAGACG CGUGUUAAAU AACA | 7364.9205 | 2453.9656 | 2453.9678 |
| diphos | ppGGGAGACG CGUGUUAAAU AACA | 7284.9547 | 2427.3103 | 2427.3105 |
| | | | | |
| mon phos | pGGGAGACG CGUGUUAAAU AACA | 7204.9879 | 2400.6547 | 2400.6563 |
| Rev cap | pm7GGGAGACG CGUGUUAAAU AACA | 7220.0035 | 2405.3240 | 2405.3271 |
| | | | | |
| Cap1 | m7GpppmGGGAGACG CGUGUUAAAU AACA | 7659.0412 | 2551.6699 | 2551.6707 |
| Cap1 triphos | pppmGGGAGACG CGUGUUAAAU AACA | 7378.9361 | 2458.6375 | not observed |
| Cap1 diphos | ppmGGGAGACG CGUGUUAAAU AACA | 7298.9702 | 2431.9822 | not observed |
| Cap1 monphos | pmGGGAGACG CGUGUUAAAU AACA | 7219.0039 | 2405.3267 | 2405.3271 |
| | | | | |
| G cap | GpppGGGAGACG CGUGUUAAAU AACA | 7630.0021 | 25423261 | 2542.3219 |
| | | | | |
| 5mC Cap0 | m7GpppGGGAGAmCG mCGUGUUAAAU AAmCA | 7687.0731 | 2561.0138 | 2561.0137 |
| 5mC uncap triphos | pppGGGAGAmCG mCGUGUUAAAU AAmCA | 7406.9685 | 2467.9816 | 2467.9686 |
| 5mC uncap diphos | ppGGGAGAmCG mCGUGUUAAAU AAmCA | 7327.0022 | 2441.3262 | 2441.327 |
| 5mC G cap | GpppGGGAGAmCG mCGUGUUAAAU AAmCA | 7672.0496 | 2556.3420 | 2556.3487 |
| | *m7G has a positive charge so an extra hydrogen must be removed to yield a ne gative charge. | | | |

See TABLE 1 for the corresponding SEQ ID NOs.

### A radiolabel free method for determining the 5' end orientation on target RNA

Cap orientation analysis: In this forty-second embodiment, once separated from the mRNA, the cap can be identified by differences in HPLC retention times. Since cap orientation is important to translation efficiency, we wanted to see if our system of cleaving and analyzing the 5' end of mRNA could also be used to determine cap orientation.

As described above, the synthesis of capped mRNA by adding a dinucleotide (m7GpppG) into the mixture of triphosphates can result in a cap with two orientations on the mRNA : m7GpppG....mRNA or Gpppm7G....mRNA. The development of anti-reverse cap analogues (ARCA) to allow correct orientation of the cap relied on several methods to assess capping orientation. Methods to study cap orientation have used 5' RNA pyrophosphohydrolase (RppH, New England Biolabs, Ipswich MA USA), tobacco acid pyrophosphatase, or other pyrophosphatases to selectively cleave off the cap. Pasquinelli AE et al., RNA 1(9):957-967 (1995); Peng ZH et al., Org. Lett. 4(2): 161-164 (2002); Stepinski J et al., RNA 7(10): 1486-1495 (2001); Jemielity J et al., RNA 9(9): 1108-1122 (2003); Grudzien E et al., RNA 10(9): 1479-1487 (2003); and Grudzien-Nogalska E et al., Methods Enzymol. 431: 203-227 (2007).

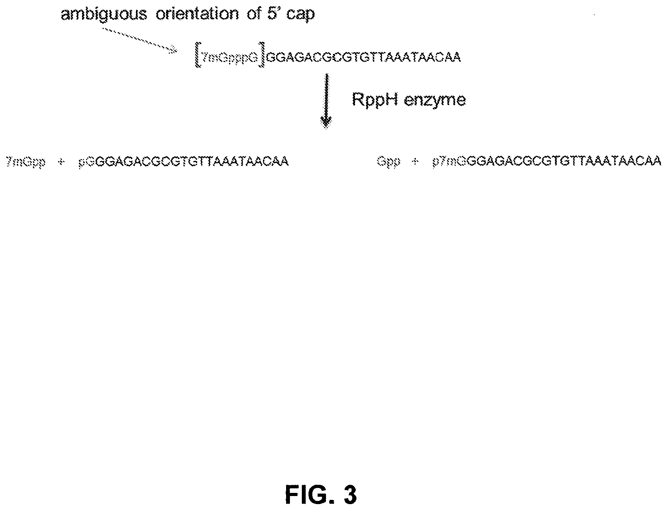

The enzymatic strategy consisted of adding RppH enzyme to the RNAse H cleaved CapO fragment and then detecting the mass difference in the product to determine the orientation of the cap. See, FIG. 3. In this embodiment, the RNase H reaction was carried out as originally described, and the beads were washed of RNAse H buffer, enzyme and unbound mRNA, but not eluted. The RppH reaction was then carried out on the bound CapO cleavage product.

In one example of a forty-third embodiment of the invention, RNAse H, RNase H reaction buffer and RNA 5'-pyrophosphohydrolase (RppH) with NEbuffer 2 were obtained from New England Biolabs (part #s M0297L and M0356S, Ipswich MA USA). For cap orientation analysis, a step was added to the method of the invention, whereby the RppH enzyme (10 units) along with its reaction buffer was added to the washed beads and reacted for one hour at room temperature. After this reaction, the beads were washed three times with deionized water before eluting.

The results from LC-MS analysis of the reaction products show the expected RNAse H cleavage fragment for the probe along with smaller peaks corresponding to the RppH reaction products (see, TABLE 2). While the RppH enzyme did not completely cleave all of the available RNase H fragment substrate as seen by the large peak at m/z 2548, enough of the product was produced to determine cap orientation. The observed peak at *m*/*z* 2401 is the expected peak for the RppH enzymatic product with the correct orientation (pGGGAGACGCGUGUUAAAUAACA) (SEQ. ID. NO.: 4) and no product for the reverse orientation at m/z 2405 was observed.

While the reverse cap species was not expected to be observed with the "post-transcriptional" capping procedure that we used to produce the mRNA, this result demonstrates the feasibility of using the RNase cleavage process to examine orientation.

### A radiolabel free method for determining capping efficiency

Uncapped spiking assays. In a forty-fourth embodiment, to determine the amount of uncapped mRNA (5' triphosphate) the assay was capable of detecting, 100 pmol of CapO mRNA was spiked with 0.5, 1, 5, 10 and 25 pmol of uncapped mRNA. The peak areas of the CapO and triphosphate ions were recorded. The background area of triphosphate present in the CapO sample alone was subtracted from the spiked samples and the areas were then plotted versus spike percentage.

Measuring *capping* efficiency. To quantify the various 5' end species and calculate the amount of capping in a given mRNA sample, we assumed that the ionization efficiency of the different cleavage species were the same. Tests with equal amounts of capped and uncapped sample gave similar responses by UV (260 nm) and ion counts, so our assumption was reasonable.

To test the linearity and reproducibility of the procedure, varying percentages of uncapped mRNA were spiked into 100 picomoles of capped 2.2K mRNA. Spiked samples were run through the procedure as separate samples, three at each percentage level. Peak areas of the most intense charge state ion, -8, for capped and uncapped species were measured and used to determine the amount of uncapped sample present. The peak area of uncapped triphosphate mRNA species present as background in the CapO sample was subtracted from the spiked samples when determining the amount of uncapped mRNA present.

Results from mixture of Cap1 and uncapped 2.2K mRNA 5' cleavage fragments. Reversed-phase chromatography easily separated the triphosphate or uncapped cleavage product from the Cap1 cleavage product. The electrospray mass spectra of the capped and uncapped RNA consisted of charge states from -9 to -3 with the -8 being the most intense. The -3 charge state ions from the Cap1 and uncapped sample (triphosphate) were roughly equivalent. Both showed a small amount of sodium and potassium adducts from the reaction buffer. In addition to the expected cleavage fragments, there were also ions corresponding to the biotin cleavage probe and ions identifying a second RNAse H cleavage site, as shown in FIG. 2 as the small arrow.

The appearance of the biotin cleavage probe was a result of harsh elution conditions that are used to release the bound 5' cleavage fragment. Part of the procedure involves drying down and re-suspending the samples, which others have reported as being problematic for oligonucleotides by Zhang G et al., Anal. Chem. 79(9): 3416-3424 (2007).

The diphosphate ion was observed in both capped and uncapped samples. It does not appear to be created by LC-MS conditions. Altering source temperatures, column temperatures and lens voltages did not affect the observed diphosphate ion.

The results showed the observed percent uncapped versus known percent uncapped mRNA for 100 pmol 2.2K mRNA spiked with uncapped triphosphate mRNA. For the data in TABLE 3, three individual samples were spiked at each percentage and analyzed. The CapO mRNA contained 0.36% uncapped triphosphate and this background level was subtracted from the spiked samples.

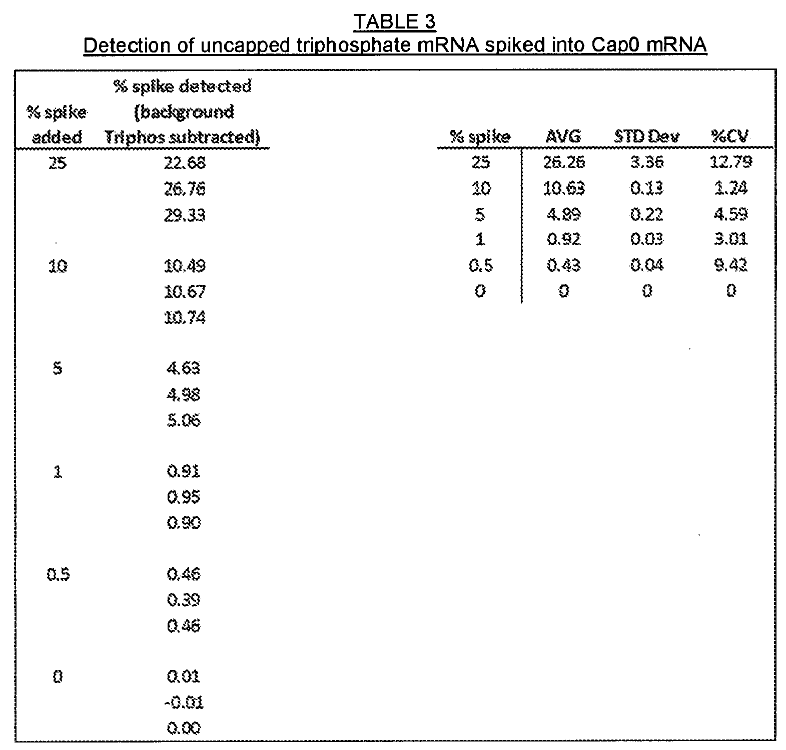

TABLE 3 shows the linear response and calculated values of the assay from 25% down to 0.5% uncapped mRNA in 100 pmols of capped mRNA. The percent coefficient of variance was above 10 for the 25% spike series.

For the tests shown in TABLE 4, 100 pmol 2.2K mRNA spiked with various amounts of uncapped triphosphate mRNA and analyzed by LC-MS. The major cleavage species identified in the tested samples are listed, along with their percentages. Percentages were calculated from the peak areas of the -8 charge state ions for each individual species.

**TABLE 4**

| **Calculated % capping species determined using selected ion peak areas for capped and uncapped species observed in IVT synthesized mRNA % capping species** | | | | | |
|---|---|---|---|---|---|
| **mRNA Information** | **% capped = m7GpppN** | **% G cap = GpppN** | **% diphosphate** = **ppN** | **% triphosphate = pppN** | **Total % Uncapped** |
| 2.2K mRNA-Pseudo U CapO | 95.3 | 2.59 | 1.77 | 0.29 | 4.7 |
| 2.2K mRNA-Pseudo U CapO | 94.9 | 2.91 | 1.93 | 0.22 | 5.1 |
| 2.2K mRNA wt CapO | 98.0 | 1.32 | 0.56 | 0.14 | 2.0 |
| 2.2K mRNA wt CapO | 97.4 | 1.60 | 0.77 | 0.23 | 2.6 |
| | | | | | |
| 2.2K mRNA *psU 5MeC* CapO | 87.8 | 7.44 | 4.15 | 0.62 | 12.2 |
| 2.2K mRNA Pseudo U-Cap1 | 97.5 | 1.38 | 0.91 | 0.25 | 2.5 |
| | | | | | |
| 9K mRNA wt CapO | 96.5 | 2.19 | 1.05 | 0.25 | 3.5 |
| 9K mRNA wt CapO | 95.4 | 3.71 | 0.87 | 0.00 | 4.6 |
| 9K mRNA wt CapO | 91.7 | 5.16 | 2.78 | 0.36 | 8.3 |
| 9K mRNA wt CapO | 95.1 | 3.94 | 0.82 | 0.12 | 4.9 |
| 9K mRNA wt CapO | 95.9 | 3.37 | 0.60 | 0.17 | 4.1 |
| | | | | | |
| | **% capped = m7GpppN** | **after capping dephosphatase reacted** | | **5' OH = N** | |
| 9K mRNA ARCA Cap 0 | 71.9 | 0.0 | 0.0 | **28.12** | **28.1** |

| | | | | | |
|---|---|---|---|---|---|
| * No CapO was observed in the Cap1 sample. | | | | | |

Each sample represents a separate IVT synthesis preparation. Total percent uncapped is determined by calculating the (peak area of all uncapped species/peak area of capped and uncapped species)*100. The mRNA capped with the ARCA procedure was treated with diphosphatase and therefore has only 5' OH as the uncapped species detected.

TABLE 5 shows the raw data for TABLE 4.

**TABLE 5**

| **Peak areas of selected ion chromatograms for capped and uncapped species detected in IVT reactions** | | | | | |
|---|---|---|---|---|---|
| **mRNA Information** | **Area Cleavage Peak Cap 0= m7GpppN** | **unmethylated G from m7G = GpppN (uncapped)** | **Area Uncapped diphosphate = ppN** | **Area Uncapped triphosphate = pppN** | **Total Area Uncapped** |
| 2.2K mRNA-Pseudo U CapO | 143,866,658 | 3,913,867 | 2,663,938 | 438,836 | 7,016,641 |
| 2.2K mRNA-Pseudo U CapO | 127,157,089 | 3,893,792 | 2,590,580 | 294,181 | 6,778,553 |
| 2.2K mRNA wt CapO | 135,857,464 | 1,835,325 | 770,040 | 187,661 | 2,793,026 |
| 2.2K mRNA wt CapO | 115,067,975 | 1,889,422 | 915,586 | 269,918 | 3,074,926 |
| | | | | | |
| 2.2K mRNA *psU 5MeC* CapO | 122,150,875 | 10,350,263 | 5,778,955 | 867,862 | 16,997,080 |
| | | | | | |
| 9K mRNA wt CapO | 10,042,471 | 227,905 | 108,761 | 26,249 | 362,915 |
| 9K mRNA wt CapO | 6,592,062 | 256,475 | 59,804 | 0 | 316,279 |
| 9K mRNA wt CapO | 14,609,106 | 822,621 | 443,386 | 57,059 | 1,323,066 |
| 9K mRNA wt CapO | 6,206,318 | 256,904 | 53,268 | 7,853 | 318,025 |
| 9K mRNA wt CapO | 7,166,897 | 252,037 | 44,698 | 12,831 | 309,566 |

| | **Area Cleavage Peak Capl = m7GpppmN** | **Area unmethylated G from m7G** = **GpppN (uncapped)** | **Area Uncapped diphosphate** = **ppN** | **Area Uncapped triphosphate = pppN** | **Total Area Uncapped*** |
|---|---|---|---|---|---|
| 2.2K mRNA Pseudo U-Cap1 | 151,688,119 | 2,141,731 | 1,411,031 | 382,468 | 3,935,230 |
| | **% capped = m7GpppN** | **after capping dephosphatase reacted** | | **5' OH=N** | |
| 9K mRNA ARCA Cap 0 | 27,070,588 | 0 | 0 | 10,592,109 | 10,592,109 |

| | | | | | |
|---|---|---|---|---|---|
| * No CapO was observed in the Cap1 sample. | | | | | |

Analysis revealed that all the studied mRNAs, regardless of size, cap type, or modified base, contained unmethylated G cap, di and triphosphates as the major uncapped species. Individual IVT synthesis reactions were largely consistent within a given set of reaction conditions. For example, all the batches of wild type (wt) 2.2K mRNAs had similar capping profiles as did all the batches of pseudouridine 2.2K mRNA and 9K wt mRNA. Capping efficiency was calculated by dividing the peak area of all the identified uncapped species by the peak area of the uncapped plus the capped species. The calculated capping efficiencies of the wild type mRNA was 98 and 95% for the 2.2K and 9K mRNA respectively.

These results are consistent with the product literature supplied with the *Vaccinia* Capping system (New England Biolabs, Ipswich MA USA), which states that capping efficiencies are expected to be close to 100%. However, we are not aware that determinations of this have been reported in the scientific literature. The mRNA capped using the ARCA system and then treated with phosphatase to remove any free phosphates on the 5' end had significantly lower capping efficiency. This result is consistent with a 70% capping efficiency with ARCA observed by Grudzien E et al., RNA 10(9): 1479-1487 (2004).

In a forty-sixth embodiment, TABLE 6 provides the data for producing a calibration curve. The peak areas are for the -8 charge state.

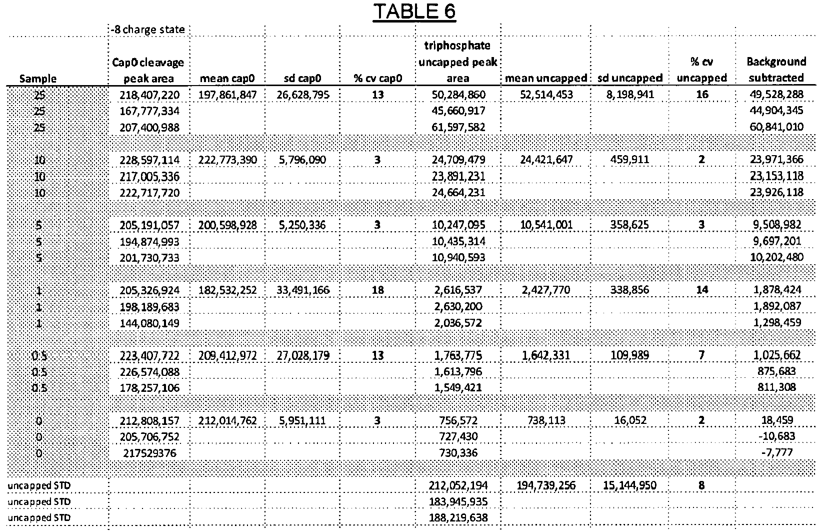

While the calibration curve was not extended past 25%, there is no reason to expect that the response would not remain linear. The size of the peak areas of a 50% mixture (as shown in FIG. 2) supports this rationale.

Analysis of IVT mRNA synthesis incorporating modified nucleosides. In a forty-seventh embodiment, the assay of the invention was used to analyze mRNA of different lengths (2.2K and 9K), as well as mRNA synthesized with Cap1, CapO and with the modified nucleotide 5-methylcytosine and pseudouridine. These two modifications were tested. Others have reported that incorporation of pseudouridine and 5-methylcytosine into mRNA results in lower immunogenicity and stability of therapeutic mRNA, but the capping efficiency of these incorporations was not reported by Kormann MS et al., Nat. Biotechnol. 29(2): 154-157 (2011); Warren L et al., Cell Stem Cell 7(5): 618-630 (2010); Karikó K et al., Immunity 23 (2):165-175 (2005); or Karikó K et al., Mol. Ther. 16(11): 1833-1840 (2008).

In general, it appears that incorporation of pseudouridine and/or 5-methylcytidine into mRNA increases the amount of uncapped mRNA present although the results are not clear for the 2.2K mRNA. Compared to wild type, the incorporation of pseudouridine into the 2.2K CapO mRNA increased the amount of uncapped species by a small amount; roughly 2.5%. However, the incorporation of pseudouridine into 2.2 K mRNA that was further methylated to Cap1 did not show an increase in uncapped species. In contrast, the incorporation of pseudouridine and 5-methylcytidine into the 9K mRNA resulted in a significant increase in uncapped mRNA over the wild type. These two nucleosides have been shown to increase the Tₘ of oligonucleotides, so it is possible that their incorporation alters the structure of the mRNA making the 5' end less accessible for capping. Davis DR et al., J. Biomol. Struct. Dyn. 15(6): 1121-1132 (1998).

RNAse H cleavage efficiency, specificity and recovery. Examination of the enzymatic steps involved in the *Vaccinia* mRNA capping gives insight into how the observed uncapped species are created. Ghosh & Lima, Wiley Interdiscip. Rev. RNA 1(1): 152-172 (2010). See also, FIG. 1.

Using the percentages or peak areas as a guide to the amount of each species present, the initial enzyme in the process (triphosphatase) works well, judging by the presence of diphosphate on the 5' end of the cleaved sequence. The next step in the process, the addition of GTP to diphosphate by guanyltransferase, is also functioning, by the observation of the G capped sequence, but the presence of remaining diphosphate is a signal that the guanlytransferase could be more efficient. Similarly, the observance of unmethylated G capped species indicates that the methylation of the terminal guanine via N7-methyltransferase is inefficient leaving unmethylated G Cap species. Observations on the Cap1 sample are interesting; unmethylated G cap was observed, but not CapO. The 2' O-ribose methyltransferase required to produce Cap1 therefore appears more efficient compared to the N7-methyltransferase.

The 48mer mRNA substrate used to determine recovery and reaction efficiency had the following 5' to 3' RNA sequence:
rGrGrGrArGrArCrGrCrGrUrGrUrUrArArArUrArArCrArArArUrCrUrCrArArCrArCrArArCrAr UrArUrArCrArArArArCrA (SEQ ID NO.: 16). The probe used in these tests contained six DNA nucleotides (see, SEQ ID NO.: 15). The probe cleaved at two adjacent positions to form two products differing by one adenosine, as shown by the arrows in FIG. 2.

Comparing ion peak areas, the longer cleavage product (CAA) was 7 to 8% of the shorter (CA) cleavage product.

In a forty-eighth embodiment, the efficiency of the RNAse H cleavage reaction and recovery of the cleavage product from the bead was tested with a 48mer RNA synthesized with the same sequence as the 5' end of the mRNA. The 48mer RNA at 200 pmol was put through the cleavage and isolation procedure without adding RNAse H and the peak area was compared to the 48mer alone pre-extraction. Recovery was found to be 94% demonstrating that little of the oligo was lost once bound to the bead. The procedure was then carried out with the RNAse H enzyme and cleavage peak areas were compared between the 48mer RNA and the 2.2 kb mRNA (both at 200 pmol). Here the mRNA cleavage peak was 58% of the 48mer, which suggests that it is either the initial binding of the cleavage probe to the mRNA and/or the RNase H cleavage reaction that is the cause of the lower recovery compared to the 48mer.

This result is not surprising, considering the size of the mRNA and the structural elements that could be interfering with the binding of the mRNA to the biotin cleavage probe.

### A radiolabel free method for detecting capping reaction impurities in an RNA preparation

In a forty-ninth embodiment, the method of the invention showed good sensitivity for detecting capping reaction impurities. Uncapped triphosphate mRNA spiked into 100 picomoles capped mRNA could be detected over the tested range of 0.5 to 25% with a linear response. The capping efficiency of several *Vaccinia* capped mRNA preparations was determined to be between 88 and 98% depending on the modification type and length of the mRNA.

### EMBODIMENTS

The following embodiments are provided by the disclosure of the invention:
1. A radiolabel free method for identifying a 5' end cap on a target ribonucleic acid (RNA), comprising the steps of:
   (a) hybridizing a nonradiolabeled tagged probe to the target RNA, wherein the nucleotide sequence of the nonradiolabeled tagged probe is complementary to the 5' end of the target RNA, thus forming a duplex polynucleotide;
   (b) treating the duplex polynucleotide with RNAse H, thus cleaving the 5' end of the target RNA and forming a duplex polynucleotide containing the 5' end of the target RNA;
   (c) isolating the duplex polynucleotide, using a surface coated substrate that is coated with a reagent that binds to the nonradiolabeled tagged probe;
   (d) removing the duplex polynucleotide from the surface coated substrate;
   (e) denaturing the duplex polynucleotide, thus producing a single-stranded fragment of the 5' end of the target RNA and the nonradiolabeled tagged probe;
   (f) isolating the single-stranded fragment of the 5' end of the target RNA;
   (g) analyzing the single-stranded fragment of the 5' end of the target RNA by liquid chromatography/mass spectrometry (LC-MS); and
   (h) identifying the 5' end cap.
2. The method of embodiment 1, wherein the target RNA is a messenger RNA (mRNA).
3. The method of embodiment 1, wherein the target RNA is a eukaryotic messenger RNA.
4. The method of embodiment 1, wherein the surface coated substrate comprises magnetic beads.
5. The method of embodiment 1, wherein the reagent on the surface coated substrate that binds to the nonradiolabeled tagged probe is selected from the group consisting of:
   (a) streptavidin, wherein the nonradiolabeled tag is biotin;
   (b) avidin, wherein the nonradiolabeled tag is biotin;
   (c) an anti-biotin antibody, wherein the nonradiolabeled tag is biotin;
   (d) an anti-digoxigenin antibody, wherein the nonradiolabeled tag is digoxigenin; and
   (e) an anti-peptide antibody, wherein the nonradiolabeled tag is a peptide.
6. The method of embodiment 1, wherein the target RNA is synthesized *in vitro.*
7. The method of embodiment 1, further determining quantitative and qualitative information on the 5' cap from the unique mass of the single-stranded fragment of the 5' end of the target RNA.
8. A radiolabel free method for determining the 5' end orientation on target RNA, comprising the steps of:
   (a) hybridizing a nonradiolabeled tagged probe to the target RNA to form a duplex polynucleotide, wherein the nucleotide sequence of the nonradiolabeled tagged probe is complementary to the 5' end of the target RNA;
   (b) treating the duplex polynucleotide with RNAse H, to cleave the 5' end of the RNA and to form a duplex polynucleotide;
   (c) isolating the duplex polynucleotide containing the 5' end of the target RNA, using magnetic beads coated with a reagent that binds to the nonradiolabeled tagged probe;
   (d) treating the duplex polynucleotide with 5' RNA pyrophosphohydrolase (RppH);
   (e) removing the duplex polynucleotide from the magnetic beads;
   (e) denaturing the duplex polynucleotide, to produce a single-stranded fragment of the 5' end of the target RNA and the nonradiolabeled tagged probe;
   (f) isolating the single-stranded fragment of the 5' end of the target RNA;
   (g) analyzing the single-stranded fragment of the 5' end of the RNA by liquid chromatography/mass spectrometry (LC-MS); and
   (h) detecting the mass difference in the product to determine the orientation of the 5' cap.
9. The method of embodiment 8, wherein the target RNA is a messenger RNA (mRNA) .
10. The method of embodiment 8, wherein the target RNA is a eukaryotic messenger RNA.
11. The method of embodiment 8, wherein the 5'end cap has a structure of formula I: 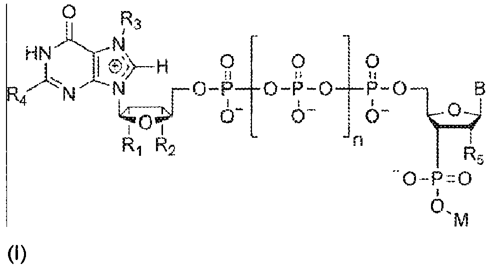 wherein,
   B is a nucleobase;
   R₁ is selected from a halogen, OH, and OCH₃;
   R₂ is selected from H, OH, and OCH₃;
   R₃ is CH₃, CH₂CH₃, CH₂CH₂CH₃ or void;
   R₄ is NH₂;
   R₅ is selected from OH, OCH₃ or a halogen;
   n is 1, 2, or 3, or a phosphorothioate; and
   M is a nucleotide of the mRNA.
12. The method of embodiment 11, wherein the nucleobase is guanine.
13. The method of embodiment 8, wherein the cap is a m⁷G cap with a structure of formula II or an unmethylated cap with a structure of formula III: 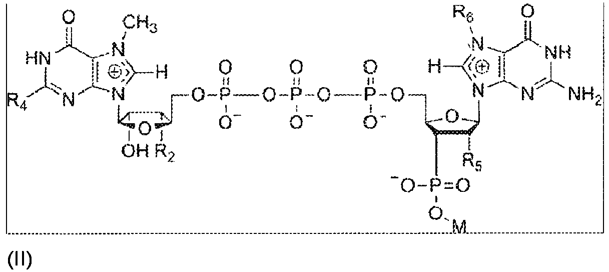 wherein,
   R₂ is H or CH₃;
   R₄ is NH₂;
   R₆ is OH or OCH₃;
   M is a nucleotide of the RNA; or 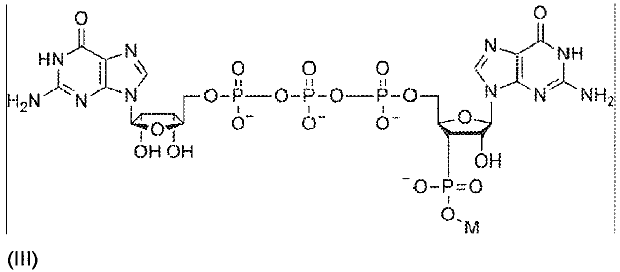
   wherein M is a nucleotide of the RNA.
14. The method of embodiment 8, wherein the target RNA is synthesized *in vitro.*
15. The method of embodiment 14, wherein the *in vitro* synthesized RNA comprises a nucleotide selected from the group consisting of Ψ (pseudouridine); m⁵C (5-methylcytidine); m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms² m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f⁵C (5-formylcytidine); m⁵Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m² ₂G (N²,N²-dimethylguanosine); m²Gm (N²,2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine); cmnm⁵s²U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N⁶,2'-O-dimethyladenosine); m3 ₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); andac⁶A (N⁶-acetyladenosine).
16. The method of embodiment 14, wherein the *in vitro* synthesized RNA comprises a nucleotide selected from the group consisting of Ψ (pseudouridine) and m⁵C (5-methylcytidine).
17. The method of embodiment 8, wherein the surface coated substrate comprises magnetic beads.
18. The method of embodiment 8, wherein the reagent on the surface coated substrate that binds to the nonradiolabeled tagged probe is a reagent selected from the group consisting of:
   (a) streptavidin, wherein the nonradiolabeled tag is biotin;
   (b) avidin, wherein the nonradiolabeled tag is biotin;
   (c) an anti-biotin antibody, wherein the nonradiolabeled tag is biotin;
   (d) an anti-digoxigenin antibody, wherein the nonradiolabeled tag is digoxigenin; and
   (e) an anti-peptide antibody, wherein the nonradiolabeled tag is a peptide.
19. A radiolabel free method for determining capping efficiency, comprising the steps of:
   (a) providing an RNA sample comprising capped RNA and uncapped RNA;
   (b) hybridizing a nonradiolabeled tagged probe to the target RNA to form a duplex polynucleotide, wherein the nucleotide sequence of the nonradiolabeled tagged probe is complementary to the 5' end of the target RNA;
   (c) treating the duplex polynucleotide with RNAse H, to cleave the 5' end of the RNA and to form a duplex polynucleotide;
   (d) isolating the duplex polynucleotide containing the 5' end of the target RNA, using magnetic beads coated with a reagent that binds to the nonradiolabeled tagged probe;
   (e) removing the duplex polynucleotide from the magnetic beads;
   (f) denaturing the duplex polynucleotide, to produce a single-stranded fragment of the 5' end of the target RNA and the nonradiolabeled tagged probe;
   (g) isolating the single-stranded fragment of the 5' end of the target RNA;
   (h) analyzing the single-stranded fragment of the 5' end of the RNA by liquid chromatography/mass spectrometry (LC-MS); and
   (i) determining relative amount of the capped and uncapped single-stranded fragments, thereby quantifying mRNA capping efficiency.
20. The method of embodiment 19, wherein the 5' end cap has a structure of formula I: 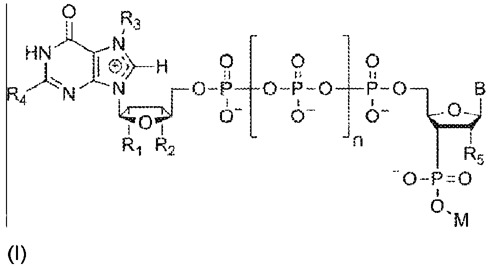 wherein,
   B is a nucleobase;
   R₁ is selected from a halogen, OH, and OCH₃;
   R₂ is selected from H, OH, and OCH₃;
   R₃ is CH₃, CH₂CH₃, CH₂CH₂CH₃ or void;
   R₄ is NH₂;
   R₅ is selected from OH, OCH₃ or a halogen;
   n is 1, 2, or 3, or a phosphorothioate; and
   M is a nucleotide of the mRNA.
21. The method of embodiment 20, wherein the nucleobase is guanine.
22. The method of embodiment 19, wherein the cap is a m⁷G cap with a structure of formula II or an unmethylated cap with a structure of formula III: 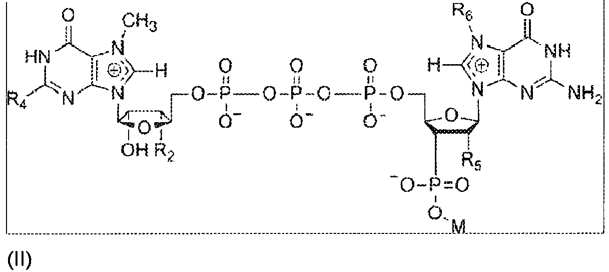 wherein,
   R₂ is H or CH₃;
   R₄ is NH₂;
   R₆ is OH or OCH₃;
   M is a nucleotide of the RNA; or 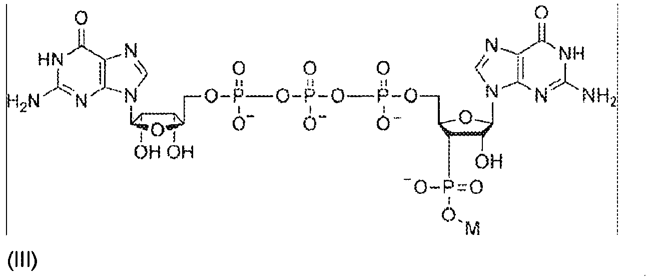
   wherein M is a nucleotide of the RNA.
23. The method of embodiment 19, wherein the target RNA is an *in vitro* transcription (IVT) reaction mixture.
24. The method of embodiment 23, wherein the IVT reaction is a *Vaccinia* capped mRNA preparation.
25. The method of embodiment 23, wherein the *in vitro* synthesized RNA comprises a nucleotide selected from the group consisting of Ψ (pseudouridine); m⁵C (5-methylcytidine); m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms² m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f⁵C (5-formylcytidine); m⁵Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m³ ₂G (N²,N²-dimethylguanosine); m²Gm (N²,2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine); cmnm⁵s²U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N⁶,2'-O-dimethyladenosine); m⁶ ₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); andac⁶A (N⁶-acetyladenosine).
26. The method of embodiment 23, wherein the *in vitro* synthesized RNA comprises a nucleotide selected from the group consisting of Ψ (pseudouridine) and m⁵C (5-methylcytidine).
27. The method of embodiment 19, wherein the surface coated substrate comprises magnetic beads.
28. The method of embodiment 19, wherein the reagent on the surface coated substrate that binds to the nonradiolabeled tagged probe is a reagent selected from the group consisting of:
   (a) streptavidin, wherein the nonradiolabeled tag is biotin;
   (b) avidin, wherein the nonradiolabeled tag is biotin;
   (c) an anti-biotin antibody, wherein the nonradiolabeled tag is biotin;
   (d) an anti-digoxigenin antibody, wherein the nonradiolabeled tag is digoxigenin; and
   (e) an anti-peptide antibody, wherein the nonradiolabeled tag is a peptide.
29. The method of embodiment 19, wherein the uncapped triphosphate mRNA is detectable over a tested range of 0.1 to 90% with a linear response.
30. The method of embodiment 19, wherein the uncapped triphosphate mRNA is detectable over a tested range of 0.5 to 25% with a linear response.
31. A radiolabel free method for detecting a capping reaction impurity in an RNA preparation, comprising the steps of:
   (a) hybridizing a nonradiolabeled tagged probe with the target RNA, wherein the nonradiolabeled tagged probe is complementary to the 5' end of the target RNA;
   (b) treating the hybridized RNA with RNAse H to cleave the 5' end of the RNA;
   (c) isolating the cleaved 5' end sequence, using magnetic beads coated with a reagent that binds to the nonradiolabeled tagged probe;
   (d) analyzing the cleaved 5' end sequence by LC-MS; and
   (e) detecting a capping reaction impurity in an RNA preparation.
32. The method of embodiment 31, further comprising the step of:
   (f) identifying a capping reaction impurity in an RNA preparation.
33. The method of embodiment 31, wherein the target RNA is synthesized *in vitro.*
34. The method of embodiment 33, wherein the *in vitro* synthesized RNA comprises a nucleotide selected from the group consisting of Ψ (pseudouridine); m⁵C (5-methylcytidine); m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms² m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f⁵C (5-formylcytidine); m⁵Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m² ₂G (N²,N²-dimethylguanosine); m²Gm (N²,2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine); cmnm⁵s²U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N⁶,2'-O-dimethyladenosine); m⁶ ₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); andac⁶A (N⁶-acetyladenosine).
35. The method of embodiment 33, wherein the *in vitro* synthesized RNA comprises a nucleotide selected from the group consisting of Ψ (pseudouridine) and m⁵C (5-methylcytidine).
36. The method of embodiment 31, wherein the surface coated substrate comprises magnetic beads.
37. The method of embodiment 31, wherein the reagent on the surface coated substrate that binds to the nonradiolabeled tagged probe is a reagent selected from the group consisting of:
   (a) streptavidin, wherein the nonradiolabeled tag is biotin;
   (b) avidin, wherein the nonradiolabeled tag is biotin;
   (c) an anti-biotin antibody, wherein the nonradiolabeled tag is biotin;
   (d) an anti-digoxigenin antibody, wherein the nonradiolabeled tag is digoxigenin; and
   (e) an anti-peptide antibody, wherein the nonradiolabeled tag is a peptide.

The contents of each of the patents and publications cited herein are incorporated by reference in their entirety.

The detailed description provided herein is to illustrate the invention, but not to limit its scope. Other variants of the invention will be readily apparent to one of ordinary skill in the biotechnological arts and are encompassed by the appended claims.

| TABLE | | |
|---|---|---|
| Nucleotide Sequences | | |
| Polynucleotide (with 5' cap) | Sequence | SEQ ID NO. |
| Cap0 | m7GpppGGGAGACGCGUGUUAAAUAACA | 1 |
| uncapped triphosphate | pppGGGAGACGCGUGUUAAAUAACA | 2 |
| uncapped diphosphate | ppGGGAGACGCGUGUUAAAUAACA | 3 |
| uncapped monophosphate (the expected peak for the RppH enzymatic product with the correct orientation) | pGGGAGACGCGUGUUAAAUAACA (gggagacgcguguuaaauaaca) | 4 |
| Rev cap | pm7GGGAGACGCGUGUUAAAUAACA | 5 |
| Cap1 | m7GpppmGGGAGACGCGUGUUAAAUAACA | 6 |
| Cap1 triphosphate | pppmGGGAGACGCGUGUUAAAUAACA | 7 |
| Cap1 diphosphate | ppmGGGAGACGCGUGUUAAAUAACA | 8 |
| Cap1 monophosphate | pmGGGAGACGCGUGUUAAAUAACA | 9 |
| G cap | GpppGGGAGACGCGUGUUAAAUAACA | 10 |
| 5mC Cap0 | m7GpppGGGAGAmCGmCGUGUUAAAUAAmCA | 11 |
| 5mC uncapped triphosphate | pppGGGAGAmCGmCGUGUUAAAUAAmCA | 12 |
| 5mC uncapped diphosphate | ppGGGAGAmCGmCGUGUUAAAUAAmCA | 13 |
| 5mC G cap | GpppGGGAGAmCGmCGUGUUAAAUAAmCA | 14 |
| Cleavage probe | TTTGTTmAmUmUmUmAmAmCmAmCmGmCmGmUmCmUmC mCmC/3BioTEG/ | 15 |
| "48mer" mRNA substrate | rGrGrGrArGrArCrGrCrGrUrGrUrUrArArArUrA rArCrArArArUrCrUrCrArArCrArCrArArCrArU rArUrArCrArArArArCrA (gggagacgcguguuaaauaacaaaucucaacacaaca uauacaaaaca) | 16 |
| *Photinus pyralis* (firefly) luciferase mRNA | cugcagaaauaacuagguacuaagcccguuugugaaaa guggccaaacccauaaauuggcaauuacaauaaagaa gcuaaaauuguggucaaacucacaaacauuuuuauuau auacauuuuaguagcugaugcuuauaaaagcaauauuu aaaucguaaacaacaaauaaaauaaaauuuaaacgaug ugauuaagagccaaagguccucuagaaaagguauuua agcaacggaauuccuuuguguuacauucuugaaugucg cucgcagugacauuagcauuccgguacuguugguaaaa uggaagacgccaaaaacauaaagaaaggcccggcgcca uucuauccucuagaggauggaaccgcuggagagcaacu gcauaaggcuaugaagagauacgcccugguuccuggaa caauugcuuuugugaguauuucugucugauuucuuucg aguuaacgaaauguucuuauguuucuuuagacagaugc acauaucgaggugaacaucacguacgcggaauacuucg aaauguccguucgguuggcagaagcuaugaaacgauau gggcugaauacaaaucacagaaucgucguaugcaguga aaacucucuucaauucuuuaugccgguguugggcgcgu uauuuaucggaguugcaguugcgcccgcgaacgacauu uauaaugaacguaagcacccucgccaucagaccaaagg gaaugacguauuuaauuuuuaaggugaauugcucaaca guaugaacauuucgcagccuaccguaguguuguuucc | 17 |
| | aaaaaggguugcaaaaaauuugaacgugcaaaaaa auuaccaauaauccagaaaauuauuaucauggauucua aaacggauuaccagggauuucagucgauguacacguuc gucacaucucaucuaccucccgguuuuaaugaauacga uuuuguaccagaguccuuugaucgugacaaaacaauug cacugauaaugaauuccucuggaucuacugguuaccu aaggguguggcccuuccgcauagaacugccugcgucag auucucgcaugccagguaugucguauaacaagagauua aguaauguugcuacacacauuguagagauccuauuuuu ggcaaucaaaucauuccggauacugcgauuuuaagugu uguuccauuccaucacgguuuggaauguuuacuacac ucggauauuugauauguggauuucgagucgucuuaaug uauagauuugaagaagagcuguuuuacgaucccuuca ggauuacaaaauucaaagugcguugcuaguaccaaccc uauuuucauucuucgccaaaagcacucugauugacaaa uacgauuuaucuaauuuacacgaaauugcuucuggggg cgcaccucuuucgaaagaagucggggaagcgguugcaa aacggugaguuaagcgcauugcuaguauuucaaggcuc uaaaacggcgcguagcuuccaucuuccagggauacgac aaggauaugggcucacugagacuacaucagcuauucug auuacacccgagggggaugauaaaccgggcgcggucgg uaaaguuguuccauuuuuugaagcgaagguuguggauc uggauaccgggaaaacgcugggcguuaaucagagaggc gaauuaugugucagaggaccuaugauuauguccgguua uguaaacaauccggaagcgaccaacgccuugauugaca aggauggauggcuacauucuggagacauagcuuacugg gacgaagacgaacacuucuucauaguugaccgcuugaa gucuuuaauuaaauacaaaggauaucagguaaugaaga uuuuuacaugcacacacgcuacaauaccuguaggugge ccccgcugaauuggaaucgauauuguuacaacacccca acaucuucgacgcgggcguggcaggucuucccgacgau gacgccggugaacuucccgccgccguuguuguuugga gcacggaaagacgaugacggaaaaagagaucguggauu acgucgccaguaaaugaauucguuuuacguuacucgua cuacaauucuuuucauaggucaaguaacaaccgcgaaa aaguugcgcggaggaguuguguuuguggacgaaguacc gaaaggucuuaccggaaaacucgacgcaagaaaaauca gagagauccucauaaaggccaagaagggcggaaagucc aaauuguaaaauguaacuguauucagcgaugacgaaau ucuuagcuauuguaauauuauaugcaaauugaugaaug guaauuuuguaauugugggucacuguacuauuuuaacg aauaauaaaaucagguauagguaacuaaaaa | |

## Claims

1. A radiolabel free method for identifying a 5' end cap on a target ribonucleic acid (RNA), comprising the steps of:
(a) hybridizing a nonradiolabeled tagged probe to the target RNA, wherein the nucleotide sequence of the nonradiolabeled tagged probe is complementary to the 5' end of the target RNA, thus forming a duplex polynucleotide;
(b) treating the duplex polynucleotide with RNAse H, thus cleaving the 5' end of the target RNA and forming a duplex polynucleotide containing the 5' end of the target RNA;
(c) isolating the duplex polynucleotide, using a surface coated substrate that is coated with a reagent that binds to the nonradiolabeled tagged probe;
(d) removing the duplex polynucleotide from the surface coated substrate;
(e) denaturing the duplex polynucleotide, thus producing a single-stranded fragment of the 5' end of the target RNA and the nonradiolabeled tagged probe;
(f) isolating the single-stranded fragment of the 5' end of the target RNA;
(g) analyzing the single-stranded fragment of the 5' end of the target RNA by liquid chromatography/mass spectrometry (LC-MS); and
(h) identifying the 5' end cap.

2. The method of claim 1, wherein the target RNA is a eukaryotic messenger RNA.

3. The method of claim 1, wherein the surface coated substrate comprises magnetic beads.

4. The method of claim 1, wherein the reagent on the surface coated substrate that binds to the nonradiolabeled tagged probe is selected from the group consisting of:
(a) streptavidin, wherein the nonradiolabeled tag is biotin;
(b) avidin, wherein the nonradiolabeled tag is biotin;
(c) an anti-biotin antibody, wherein the nonradiolabeled tag is biotin;
(d) an anti-digoxigenin antibody, wherein the nonradiolabeled tag is digoxigenin; and
(e) an anti-peptide antibody, wherein the nonradiolabeled tag is a peptide.

5. A radiolabel free method for determining the 5' end orientation on target RNA, comprising the steps of:
(a) hybridizing a nonradiolabeled tagged probe to the target RNA to form a duplex polynucleotide, wherein the nucleotide sequence of the nonradiolabeled tagged probe is complementary to the 5' end of the target RNA;
(b) treating the duplex polynucleotide with RNAse H, to cleave the 5' end of the RNA and to form a duplex polynucleotide;
(c) isolating the duplex polynucleotide containing the 5' end of the target RNA, using magnetic beads coated with a reagent that binds to the nonradiolabeled tagged probe;
(d) treating the duplex polynucleotide with 5' RNA pyrophosphohydrolase (RppH);
(e) removing the duplex polynucleotide from the magnetic beads;
(f) denaturing the duplex polynucleotide, to produce a single-stranded fragment of the 5' end of the target RNA and the nonradiolabeled tagged probe;
(g) isolating the single-stranded fragment of the 5' end of the target RNA;
(h) analyzing the single-stranded fragment of the 5' end of the RNA by liquid chromatography/mass spectrometry (LC-MS); and
(i) detecting the mass difference in the product to determine the orientation of the 5' cap.

6. The method of claim 5, wherein the target RNA is synthesized *in vitro.*

7. The method of embodiment 33, wherein the *in vitro* synthesized RNA comprises a nucleotide selected from the group consisting of Ψ (pseudouridine); m⁵C (5-methylcytidine); m⁵U (5-methyluridine); m⁶A (N⁶-methyladenosine); s²U (2-thiouridine); Um (2'-O-methyl-U; 2'-O-methyluridine); m¹A (1-methyladenosine); m²A (2-methyladenosine); Am (2'-O-methyladenosine); ms²m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²i⁶A (2-methylthio-N⁶-(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine); s²C (2-thiocytidine); ac⁴C(N⁴-acetylcytidine); f⁵C (5-formylcytidine); m⁵Cm (5,2'-O-dimethylcytidine); ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m² ₂G (N²,N²-dimethylguanosine); m²Gm (N²,2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine); D (dihydrouridine); m⁵Um (5,2'-O-dimethyluridine); s⁴U (4-thiouridine); m⁵s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine); mcm⁵s²U (5-methoxycarbonylmethyl-2-thiouridine); nm⁵s²U (5-aminomethyl-2-thiouridine); mnm⁵U (5-methylaminomethyluridine); mnm⁵s²U (5-methylaminomethyl-2-thiouridine); mnm⁵se²U (5-methylaminomethyl-2-selenouridine); ncm⁵U (5-carbamoylmethyluridine); ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine); cmnm⁵s²U (5-carboxymethylaminomethyl-2-thiouridine); m⁶ ₂A (N⁶,N⁶-dimethyladenosine); Im (2'-O-methylinosine); m⁴C(N⁴-methylcytidine); m⁴ Cm (N⁴,2'-O-dimethylcytidine); hm⁵C (5-hydroxymethylcytidine); m³U (3-methyluridine); cm⁵U (5-carboxymethyluridine); m⁶Am (N⁶,2'-O-dimethyladenosine); m⁶ ₂Am (N⁶,N⁶,O-2'-trimethyladenosine); m^{2,7}G (N²,7-dimethylguanosine); m^{2,2,7}G (N²,N²,7-trimethylguanosine); m³Um (3,2-O-dimethyluridine); m⁵D (5-methyldihydrouridine); f⁵Cm (5-formyl-2'-O-methylcytidine); m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); andac⁶A (N⁶-acetyladenosine).

8. The method of claim 5, wherein the *in vitro* synthesized RNA comprises a nucleotide selected from the group consisting of Ψ (pseudouridine) and m⁵C (5-methylcytidine).

9. A radiolabel free method for determining capping efficiency, comprising the steps of:
(a) providing an RNA sample comprising capped RNA and uncapped RNA;
(b) hybridizing a nonradiolabeled tagged probe to the target RNA to form a duplex polynucleotide, wherein the nucleotide sequence of the nonradiolabeled tagged probe is complementary to the 5' end of the target RNA;
(c) treating the duplex polynucleotide with RNAse H, to cleave the 5' end of the RNA and to form a duplex polynucleotide;
(d) isolating the duplex polynucleotide containing the 5' end of the target RNA, using magnetic beads coated with a reagent that binds to the nonradiolabeled tagged probe;
(e) removing the duplex polynucleotide from the magnetic beads;
(f) denaturing the duplex polynucleotide, to produce a single-stranded fragment of the 5' end of the target RNA and the nonradiolabeled tagged probe;
(g) isolating the single-stranded fragment of the 5' end of the target RNA;
(h) analyzing the single-stranded fragment of the 5' end of the RNA by liquid chromatography/mass spectrometry (LC-MS); and
(i) determining relative amount of the capped and uncapped single-stranded fragments, thereby quantifying mRNA capping efficiency.

10. The method of claim 9, wherein the target RNA is an *in vitro* transcription (IVT) reaction mixture.

11. The method of claim 10, wherein the IVT reaction is a *Vaccinia* capped mRNA preparation.

12. The method of claim 9, wherein the uncapped triphosphate mRNA is detectable over a tested range of 0.1 to 90% with a linear response.

13. The method of claim 9, wherein the uncapped triphosphate mRNA is detectable over a tested range of 0.5 to 25% with a linear response.

14. A radiolabel free method for detecting a capping reaction impurity in an RNA preparation, comprising the steps of:
(a) hybridizing a nonradiolabeled tagged probe with the target RNA, wherein the nonradiolabeled tagged probe is complementary to the 5' end of the target RNA;
(b) treating the hybridized RNA with RNAse H to cleave the 5' end of the RNA;
(c) isolating the cleaved 5' end sequence, using magnetic beads coated with a reagent that binds to the nonradiolabeled tagged probe;
(d) analyzing the cleaved 5' end sequence by LC-MS; and
(e) detecting a capping reaction impurity in an RNA preparation.

15. The method of claim 14, wherein the target RNA is synthesized *in vitro.*
